# EUROPÄISCHE PATENTSCHRIFT

(11) **EP 0 653 415 B1**
(45) Veröffentlichungstag und Bekanntmachung des Hinweises auf die Patenterteilung: **24.06.1998**
(21) Anmeldenummer: 94117050.8
(22) Anmeldetag: 28.10.1994
(51) Int. Cl.: C07C 69/618, C07C 327/26, C07C 327/36, A61K 31/22, A61K 31/265

(54) **(Aryl-[1'(aryl)-alkyl]-methyliden)- bzw. (Aryl-[1'-(aryl)-alkyl]-methyl)-dicarbonsäure-alkylester bzw. ihre Thiocarbonsäureesteranaloga, Verfahren zu deren Herstellung und ihre Verwendung**
(Aryl-[1'-(aryl)-alkyl]-methylidene)- or (aryl-[1'(aryl)alkyl]-methyl)-dicarboxylic acid alkyl ester or their thiocarboxylic acid esters, process for their preparation and their use
Alkylesters d'acides (aryle-[1'-(aryle)-alkyle]-méthylidène) ou (aryle-[1'-(aryle)-alkyle]-méthyle) dicarboxyliques ou leurs analogues thiocarboxyliques et procédé pour leur préparation et leur utilisation

(30) Priorität: 28.10.1993 DE 4336889
(43) Veröffentlichungstag der Anmeldung: 17.05.1995
(73) Patentinhaber: Thiemann Arzneimittel GmbH, D-45731 Waltrop (DE); Laboratoires BIOCODEX S.A., 92126 Montrouge Cédex (FR)
(72) Erfinder: Tilman Friedland, 44534 Lünen (DE); Dr. Jürgen Seifert, 24113 Kiel (DE)
(74) Vertreter: Beszédes, Stephan G., Dr.

(56) Entgegenhaltungen:
- EP-A- 0 195 097
- STN-Information Service, file: REG RN= 77573-37-6

## Beschreibung

Die Erfindung betrifft Dicarbonsäurealkylester und ihre Thiocarbonsäureesteranaloga zur Verwendung als Arzneimittel, ein Verfahren zur Herstellung derselben und ihre Verwendung.

Der Erfindung liegt die Aufgabe zugrunde, Dicarbonsäurealkylester beziehungsweise ihre Thiocarbonsäureesteranaloga mit überlegenen pharmakologischen, insbesondere antidiarrhoischen antibiotischen, antimykotischen, immunsuppressiven und tumorhemmenden, Eigenschaften zur Verwendung als Arzneimittel, insbesondere Antidiarrhoika, Antibiotika, Antimykotika, Immunsuppressiva und Antitumormittel, ein Verfahren zu deren Herstellung und ihre Verwendung zu schaffen.

Gegenstand der Erfindung sind {Aryl-[1'-(aryl)-alkyl]--methyliden}- bzw. {Aryl-[1'-(aryl)-alkyl]-methyl}-dicarbonsäurealkylester bzw. ihre Thiocarbonsäureesteranaloga der allgemeinen Formel 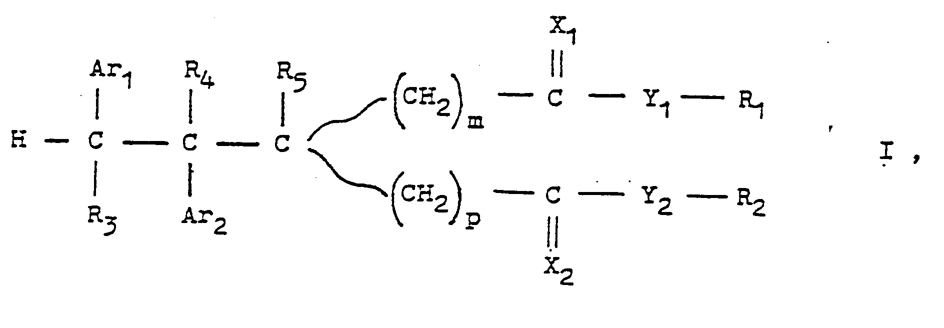 worin
- Ar₁ und Ar₂: unabhängig voneinander für Arylreste stehen,
- R₁: ein Wasserstoffatom oder einen Alkylrest mit 1 bis 5 Kohlenstoffatom(en) darstellt,
- R₂: einen Alkylrest mit 1 bis 5 Kohlenstoffatom(en) bedeutet,
- R₃: einen Alkylrest mit 1 bis 6 Kohlenstoffatom(en) darstellt,
- R₄ und R₅: für Wasserstoffatome stehen oder zusammen eine weitere Bindung bedeuten,
- X₁ und X₂: unabhängig voneinander für Sauerstoffbeziehungsweise Schwefelatome stehen,
- Y₁ und Y₂: unabhängig voneinander Sauerstoff- beziehungsweise Schwefelatome bedeuten,
- m und p: unabhängig voneinander ganze Zahlen von 1 bis 8 sind und
- die Wellenlinien: das Erfassen sowohl der E- als auch der Z-Isomere sowie deren Mischungen versinnbildlichen,
einschließlich ihrer Z- und E-Isomere und optisch aktiven Enantiomere und deren Mischungen zur Verwendung als Arzneimittel.

Der in diesem Text verwendete Ausdruck "Thiocarbonsäureesteranaloga" erfaßt sowohl die Verbindungen, bei welchen in den Estergruppen je 2 Schwefelatome statt 2 Sauerstoffatome vorliegen, als auch diejenigen, welche jeweils 1 Schwefelatom und 1 Sauerstoffatom aufweisen.

Vorzugsweise sind die Arylreste, für welche Ar₁ und Ar₂ stehen, Phenyl- beziehungsweise Naphthylreste, insbesordere die ersteren.

Es ist auch bevorzugt, daS die Alkylreste, für welche R₁ stehen kann und R₂ steht, solche mit 1 bis 4, insbesondere 1 oder 2, ganz besonders 1, Kohlenstoffatom(en) sind.

Ferner ist bevorzugt, daS der Alkylrest, für den R₃ steht, ein solcher mit 1 bis 4, insbesondere 1 oder 2, ganz besonders 1, Kohlenstoffatom(en) ist.

Weiterhin ist bevorzugt, daß m und p 1 bis 6, insbesondere 1 bis 4, ganz besonders 1 oder 2, sind.

Eine besonders bevorzugte erfindungsgemäße Verbindung ist 3-{Phenyl-[1'-(phenyl)-ethyl]-methyliden}-glutarsäuremonomethylester der Formel 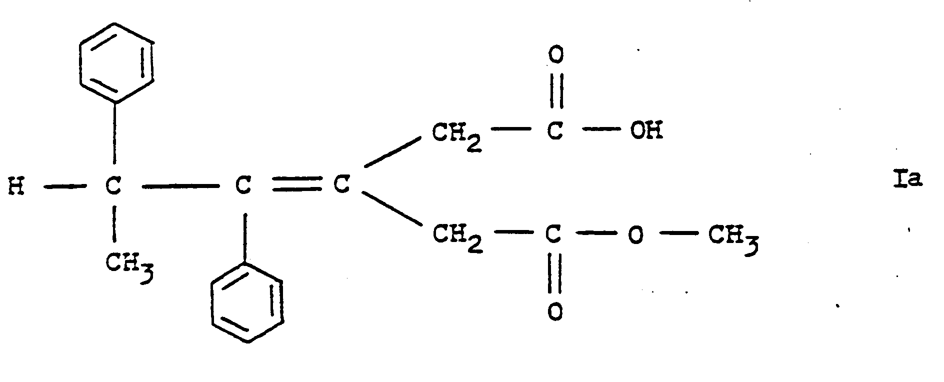 einschließlich seiner E- und Z-Isomere und optisch aktiven Enantiomere und daren Mischungen.

Gegenstand der Erfindung ist auch ein Verfahren zur Herstellung der erfindungsgemäßen Verbindungen, welches dadurch gekennzeichnet ist, daß
a) 1,2-Di-(aryl)-alkan-1-one der allgemeinen Formel 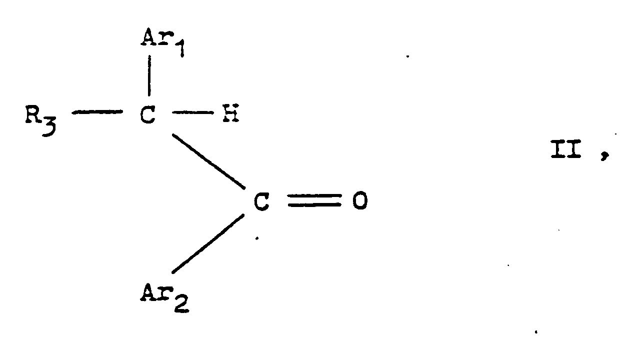 worin
   - Ar₁ , Ar₂ und R₃: die oben angegebenen Bedeutungen haben,
   mit Oxo-dicarbonsäuredialkylestern der allgemeinen Formel 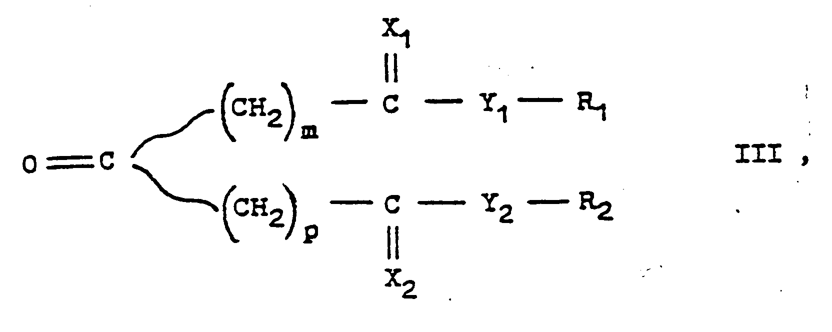 worin
   - X₁ , X₂ Y₁ , Y₂ , m und p sowie die Wellenlinien: die oben angegebenen Bedeutungen haben
   und
   - R₁ und R₂: für Alkylreste mit 1 bis 5 Kohlenstoffatom(en) stehen,
   reduktiv zu {Aryl-[1'-(aryl)-alkyl]-methyliden}-dicarbonsäuredialkylestern bzw. ihren Thiocarbonsäureesteranaloga der allgemeinen Formel I, in welcher R₁ und R₂ für Alkylreste stehen und R₄ und R₅ zusammen eine weitere Bindung darstellen, gekuppelt werden und gegebenenfalls diese letzteren regioselektiv zu {Aryl-[1'-(aryl)-alkyl]-methyliden}-dicarbonsäuremonoalkylestern bzw. ihren Thiocarbonsäureesteranaloga der allgemeinen Formel I, in welcher R₁ für ein Wasserstoffatom steht und R₂ einen Alkylrest bedeutet und R₄ und R₅ zusammen eine weitere Bindung darstellen, verseift werden oder/und die erhaltenen {Aryl-[1'-(aryl)-alkyl]-methyliden}-dicarbonsäuredialkylester bzw. -monoalkylester bzw. ihre Thiocarbonsäureesteranaloga der allgemeinen Formel I, in welcher R₄ und R₅ zusammen eine Doppelbindung darstellen, zu den entsprechenden {Aryl-[1'-(aryl)-alkyl]-methyl}-dicarbonsäuredialkylestern bzw. -monoalkylestern bzw. ihren Thiocarbonsäureesteranaloga der allgemeinen Formel I, in welcher R₄ und R₅ Wasserstoffatome bedeuten, hydriert werden
   oder
b) 1,2-Di(aryl)-alkan-1-one der allgemeinen Formel 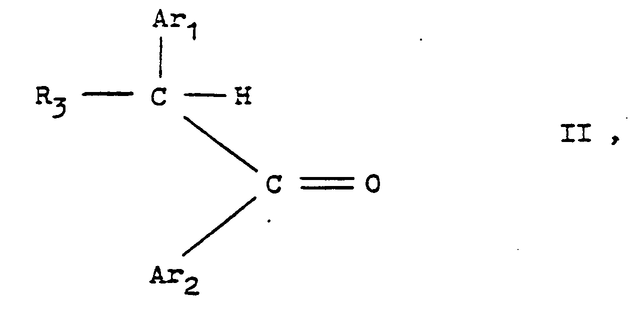 worin
   - Ar₁ , Ar₂ und R₃: die oben angegebenen Bedeutungen haben,
   mit Dicarbonsäuredialkylestern bzw. Thiocarbonsäuredialkylesteranaloga derselben der allgemeinen Formel 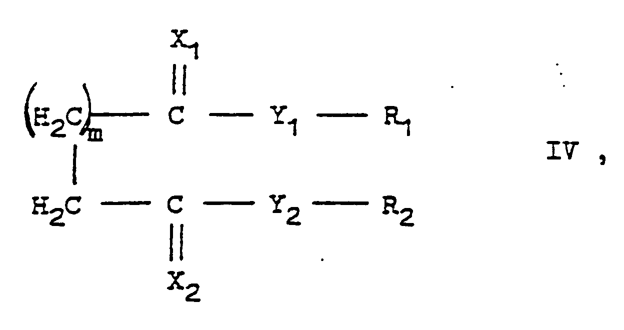 worin
   - X₁ , X₂ , Y₁ , Y₂ und m: die oben angegebenen Bedeutungen haben
   und
   - R₁ und R₂: für Alkylreste mit 1 bis 5 Kohlenstoffatom(en) stehen,
   der Stobbe-Kondensation zu 2-{Aryl-[1'-(aryl)-alkyl]-methyliden}-dicarbonsäuremonoalkylestern bzw. ihren Thiocarbonsäuremonoalkylesteranaloga der allgemeinen Formel 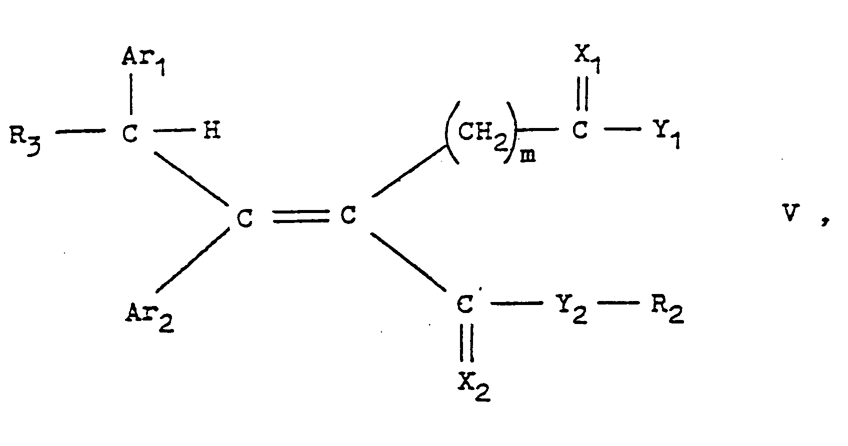 worin
   - Ar₁ , Ar₂ , R₃ , X₁ , X₂, Y₁ , Y₂ und m: die oben angegebenen Bedeutungen haben
   und
   - R₂: für einen Alkylrest mit 1 bis 5 Kohlenstoffatom(en) steht,
   unterworfen werden, diese letzteren selektiv zu 2-{Aryl-[1'-(aryl)-alkyl]-methyliden}-ω-(hydroxy)-carbonsäurealkylestern bzw. ihren Mercapto-, Thiocarbonsäurealkylester- bzw. Mercaptothiocarbonsäurealkylesteranaloga der allgemeinen Formel 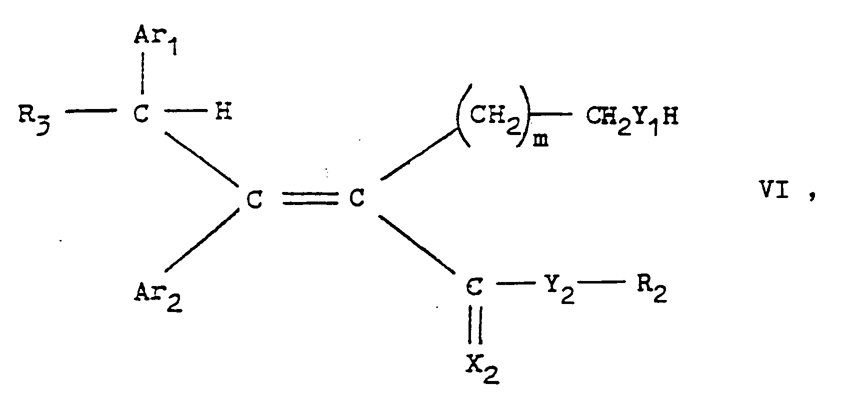 worin
   - Ar₁ , Ar₂ , R₃ , X₂ , Y₁ , Y₂ und m: die oben angegebenen Bedeutungen haben
   und
   - R₂: für einen Alkylrest mit 1 bis 5 Kohlenstoffatom(en) steht,
   reduziert werden, diese letzteren in die entsprechenden an der Gruppe CH₂Y₁- durch eine Trialkylsilylgruppe geschützten Verbindungen der allgemeinen Formel 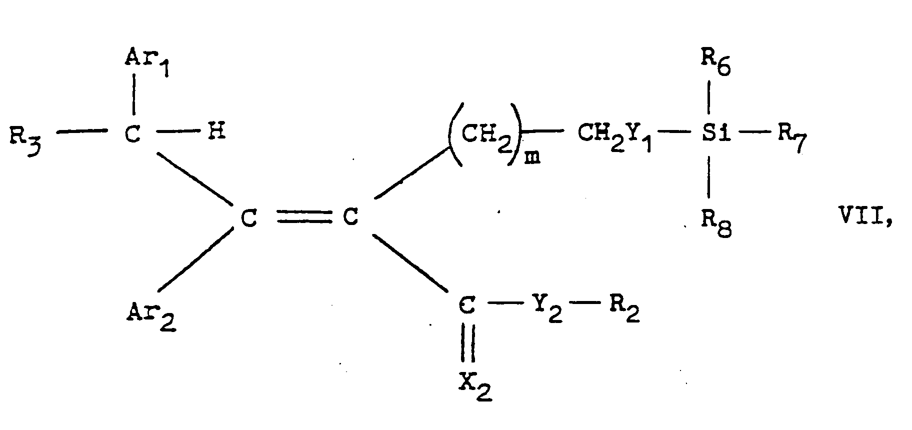 worin
   - Ar₁ , Ar₂ , R₃ , X₂ , Y₁ , Y₂ und m: die oben angegebenen Bedeutungen haben
   und
   - R₂, R₆ , R₇ und R₈: unabhängig voneinander für Alkylreste mit 1 bis 5 Kohlenstoffatom(en) stehen,
   überführt werden, an der Alkoxycarbonylgruppe bzw. ihren Thioanaloga der letzteren Verbindungen eine Kettenverlängerung um 1 Kohlenstoffatom zu 3-{Aryl-[1'-(aryl)-alkyl]-methyliden}-ω-(trialkylsilyloxy)-carbonsäurealkylestern bzw. ihren Mercapto-, Thiocarbonsäurealkylester- bzw. Mercaptothiocarbonsäurealkylesteranaloga der allgemeinen Formel 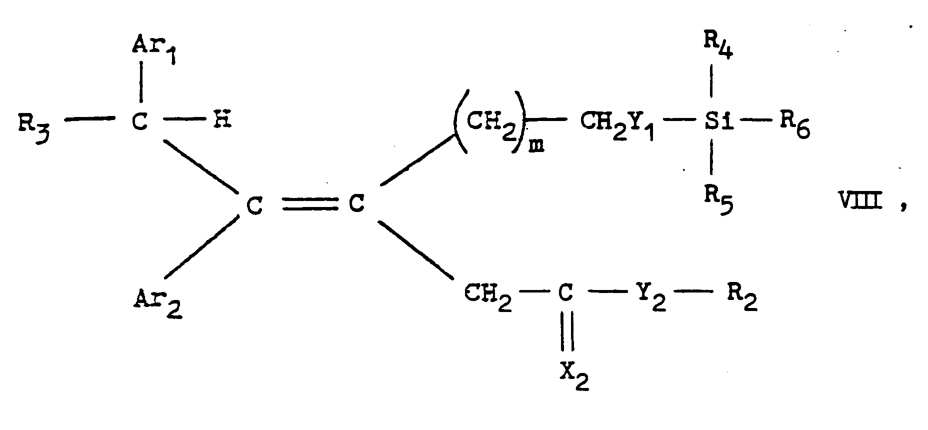 worin
   - Ar₁ , Ar₂ , R₃ , X₂ , Y₁ , Y₂ und m: die oben angegebenen Bedeutungen haben
   und
   - R₂, R₆ , R₇ und R₈: unabhängig voneinander für Alkylreste mit 1 bis 5 Kohlenstoffatom(en) stehen,
   vorgenommen und gegebenenfalls durch Wiederholen der Kettenverlängerungsumsetzung weitere Kettenverlängerungen [um 1 oder mehr weitere Kohlenstoffatom(e)] durchgeführt werden, die letzteren durch Abspalten der Trialkylsilylgruppe in {Aryl-[1'-(aryl)-alkyl]-methyliden}-ω-(hydroxy)-carbonsäurealkylester bzw. ihre Mercapto-, Thiocarbonsäurealkylester- bzw. Mercaptothiocarbonsäurealkylesteranaloga der allgemeinen Formel 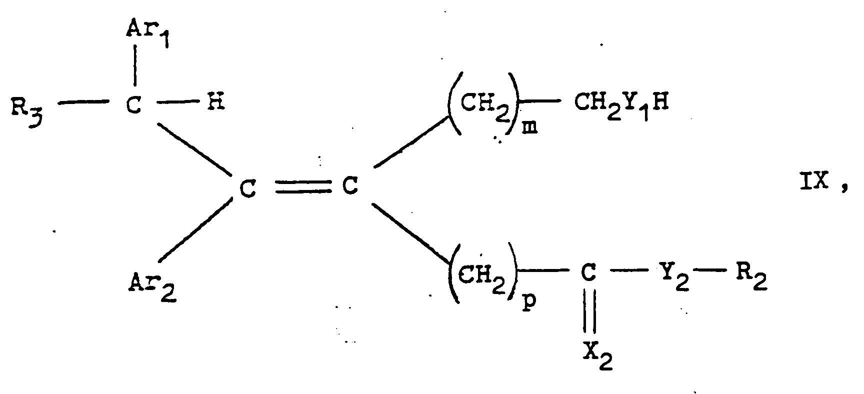 worin
   - Ar₁ , Ar₂ , R₃ , X₂ , Y₁ , Y₂ , m und p: die oben angegebenen Bedeutungen haben
   und
   - R₂, R₆ , R₇ und R₈: unabhängig voneinander für Alkylreste mit 1 bis 5 Kohlenstoffatom(en) stehen,
   überführt werden und die letzteren oxydiert werden sowie gegebenenfalls die erhaltenen {Aryl-[1'-(aryl)-alkyl]-methyliden}-dicarbonsäuredialkylester bzw. -monoalkylester bzw. ihre Thiocarbonsäureesteranaloga der allgemeinen Formel I, in welcher R₄ und R₅ zusammen eine Doppelbindung darstellen, zu den entsprechenden {Aryl-[1'-(aryl)-alkyl]-methyl}-dicarbonsäuredialkylestern bzw. -monoalkylestern bzw. ihren Thiocarbonsäureesteranaloga der allgemeinen Formel I, in welcher R₄ und R₅ Wasserstoffatome bedeuten, hydriert werden, worauf gegebenenfalls die erhaltenen {Aryl-[1'-(aryl)-alkyl]-methyliden}- bzw. {Aryl-[1'-(aryl)-alkyl]-methyl}-dicarbonsäurealkylester bzw. ihre Thiocarbonsäureesteranaloga der allgemeinen Formel I in ihre Isomere getrennt werden oder die in Form von Isomeren erhaltenen {Aryl-[1'-(aryl)-alkyl]-methyliden}- bzw. {Aryl-[1'-(aryl)-alkyl]-methyl}-dicarbonsäurealkylester bzw. ihre Thiocarbonsäureesteranaloga der allgemeinen Formel I in isomere Mischungen überführt werden.

Das reduktive Kuppeln bei der Variante a) des erfindungsgemäßen Verfahrens kann beispielsweise durch die Mc Murry-Reaktion (B. P. Mundy, M. G. Ellerd, Name Reactions and Reagents in Organic Syntheses, Wiley, New york, 1988, Seiten 140 bis 141; J. E. Mc Murry, M. P. Fleming, K. L. Kees, L. R. Krepski, J. Org. Chem. 1978, 43, 3 255 bis 3 266) durchgeführt werden. Vorteilhaft wird als Reduktionsmittel Titantrichlorid/Lithium, vorzugsweise in 1,2-Di(methoxy)-ethan, eingesetzt.

Die regioselektive Verseifung der Variante a) des erfindungsgemäßen Verfahrens wird zweckmäßig alkalisch, wie mit einer wäßrigen oder wäßrig-alkoholischen Alkalihydroxydlösung, beispielsweise Kaliumhydroxydlösung, Alkalicarbonatlösung oder Ammoniaklösung, vorzugsweise in einer Menge von 0,8 bis 1,2 Äquivalenten, insbesondere etwa 1 Äquivalent, durchgeführt.

Die Stobbe-Kondensation bei der Variante b) des erfindungsgemäßen Verfahrens kann beispielsweise gemäß der Verfahrensweise von B. P. Mundy, M. G. Ellerd, Name Reactions and Reagents in Organic Syntheses, Wiley, New York, 1988, Seiten 204 bis 205 oder W. S. Johnson, G. H. Daub, Organic Reactions, 1951, 6, Seiten 1 bis 73 durchgeführt werden. Vorzugsweise wird sie in Gegenwart von Alkalimetallhydriden, wie Natriumhydrid, vorteilhaft in aromatischen Kohlenwasserstoffen, wie Benzol, vorgenommen.

Es ist vorteilhaft, die selektive Reduktion (N. M. Yoon, C. S. Pak, H. C. Brown, S. Krishnamurthy, T. P. Stocky, J. Org. Chem. 1973, 38, Seiten 2 786 bis 2 792) der Variante b) des erfindungsgemäßen Verfahrens mit einem Hydrid durchzuführen. Bevorzugt wird Borwasserstoff, zweckmäßig in Tetrahydrofuran, eingesetzt.

Als Trialkylsilylgruppe wird bei der Variante b) des erfindungsgemäßen Verfahrens vorteilhaft eine solche, bei welcher eines von R₆ , R₇ und R₈ für einen tert. Alkylrest, vorzugsweise tert.Butylrest, steht und die anderen beiden n-Alkylreste, vorzugsweise mit 1 oder 2 Kohlenstoffatom(en), insbesondere Methylreste, bedeuten, eingeführt. Als Trialkylsilylierungsmittel (S. K. Massad, L. D. Hawkins, D. C. Baker, J. Org. Chem. 1983, 48, Seiten 5 180 bis 5 182) wird vorzugsweise ein tert.Butyldimethylhalogensilan, insbesondere tert. Butyldimethylchlorsilan, verwendet. Zweckmäßig wird die Umsetzung in Gegenwart von 4-Dimethylaminopyridin und einem Trialkylamin, wie Triethylamin, vorteilhaft in Tetrahydrofuran, durchgeführt.

Die Kettenverlängerung um 1 Kohlenstoffatom an der Alkoxycarbonylgruppe bei der Variante b) des erfindungsgemäßen Verfahrens kann nach bekannten Verfahrensweisen, zum Beispiel durch eine Arndt-Eistert-Reaktion (H. Beyer, W. Walter, Lehrbuch der Organischen Chemie, 21. Auflage, Hirzel Verlag, Stuttgart, 1988, Seiten 254 bis 255) oder mit Dibrommethan (Organic Syntheses 1992, 71, Seiten 146 bis 157) durchgeführt werden. Analoges gilt für ihre Wiederholung. Vorteilhaft wird sie mit einem Dihalogenalkan, vorzugsweise Dibrommethan, durchgeführt. Dabei ist es bevorzugt, das Dihalogenalkan in Form eines, zweckmäßig in situ erzeugten, Alkalidihalogenalkanes, insbesondere Lithiumdihalogenalkanes, ganz besonders von Lithiumdibrommethan, einzusetzen. Ferner ist es bevorzugt, die Umsetzung in Gegenwart eines nachfolgend zugesetzten Gemisches aus einem Alkalihexaalkyldisilazan, insbesondere Alkalihexamethyldisilazan, wie Lithimhexamethyldisilazan, und einem Alkalimetallalkylat, insbesondere Alkalimetallethylat, wie Lithiummethylat, und eines danach zugesetzten Alkylalkalis, insbesondere Butylalkalis, wie Butyllithiums, beispielsweise von sek. Butyllithium, vorzunehmen.

Vorteilhaft wird das Abspalten der Trialkylsilylgruppe (E. J. Corey, A. Venkateswarlu, J. Am. Chem. Soc. 1972, 94, Seiten 6 190 bis 6 191) der Variante b) des erfindungsgemäßen Verfahrens mit einem Tetraalkylammoniumfluorid, vorzugsweise Tetrabutylammoniumfluorid, zweckmäßig in Tetrahydrofuran, durchgeführt.

Vorteilhaft wird die Oxydation (Autorenkollektiv, Organikum, 18. Auflage, Deutscher Verlag der Wissenschaften, Berlin, 1990; L. F. Tietze, Th. Eicher, Reaktionen und Synthesen im organisch-chemischen Praktikum und Forschungslaboratorium, 2. Auflage, Thieme Verlag, Stuttgart, 1991) der Verbindungen der allgemeinen Formel IX der Variante b) des erfindungsgemäßen Verfahrens mit einem Alkalimetalldichromat, wie Natriumdichromat, zweckmäßig in saurer, vorzugsweise schwefelsaurer, Lösung, vorgenommen.

Das gegebenenfalls erfolgende Hydrieren der erhaltenen Verbindungen der allgemeinen Formel I der Variante b) des erfindungsgemäßen Verfahrens kann in an sich bekannter Weise durchgeführt werden.

Der Weg der Variante b) des erfindungsgemäßen Verfahrens hat den Vorteil, daß nach selektivem Schutz der freien Carboxylgruppe nicht nur eine Kettenverlängerung um 1 Kohlenstoffatom an der Alkoxycarbonylgruppe in an sich bekannter Weise durchführbar ist, sondern zahlreiche erfindungsgemäße Analoga mit variierten Kettenlängen zugänglich sind.

Durch beide Varianten des erfindungsgemäßen Verfahrens können alle Stereoisomere der erfindungsgemäßen {Aryl-[1'-(aryl)-alkyl]-methyliden}- bzw. {Aryl-[1'-(aryl)-alkyl]-methyl}}-dicarbonsäurealkylester bzw. ihrer Thiocarbonsäureesteranaloga mit den möglichen E,Z- und R,S-Konfigurationen erhalten werden.

Für die Herstellung der erfindungsgemäßen Verbindung 3-{Phenyl-[1'-(phenyl)-ethyl]-methyliden}-glutarsäuremonomethylester der Formel 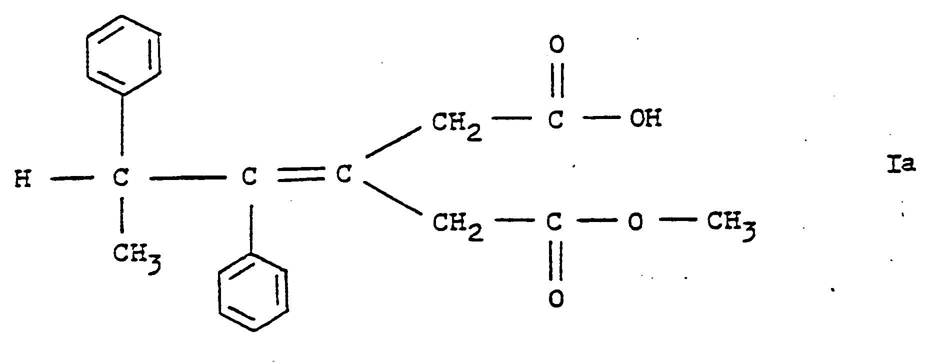 kann als Ausgangssubstanz 1,2-Di-(phenyl)-propan-1-on der Formel 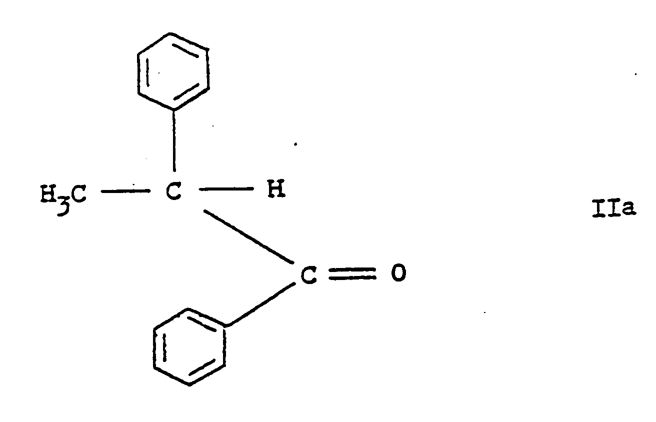 eingesetzt werden. Von dieser sind sowohl das racemische Gemisch als auch beide Enantiomere bekannt.

Nach der Variante a) des erfindungsgemäßen Verfahrens wird diese Verbindung mit 3-(Oxo)-glutarsäuredimethylester der Formel 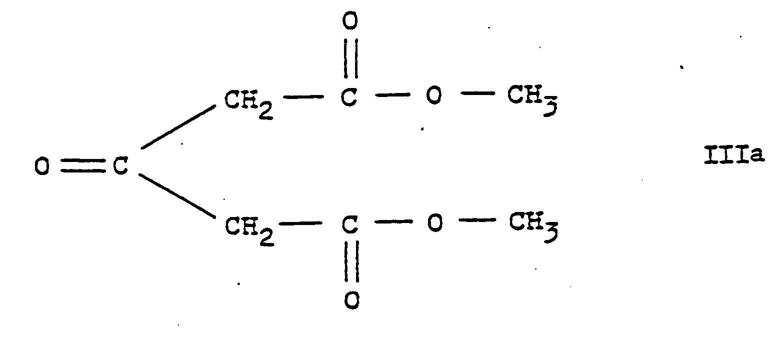 direkt reduktiv, beispielsweise durch die Mc Murry-Reaktion, zu 3-{Phenyl-[1'-(phenyl)-ethyl]-methyliden-glutarsäuredime-thylester gekuppelt. Eine regioselektive Verseifung des letzteren führt zum 3-{Phenyl-[1'-(phenyl)-ethyl]-methyliden}-glutarsäuremonomethylester der Formel Ia.

Nach der Variante b) des erfindungsgemäßen Verfahrens wird das 1,2-Di-(phenyl)-propan-1-on der Formel IIa der Stobbe-Kondensation mit Bernsteinsäuredimethylester der Formel 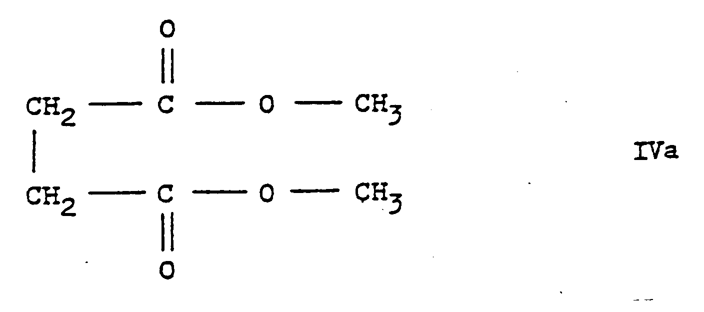 zu 3-(Methoxy)-carbonyl-4,5-di-(phenyl)-3-hexensäure 〈2-{Phenyl-[1'-(phenyl)-ethyl]-methyliden}-bernsteinsäuremonomethylester〉 der Formel 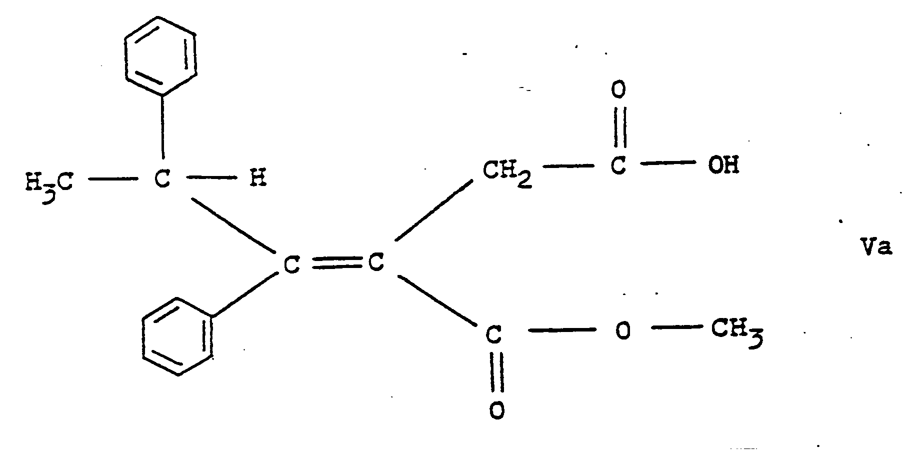 unterworfen. Dieser wird zu 2-[2'-(Hydroxy)-ethyl]-3,4-di-[phenyl]-2-pentensäuremethylester 〈2-{Phenyl-[1'-(phenyl)-ethyl]-methyliden}-4-{hydroxy}-buttersäuremonomethylester der Formel 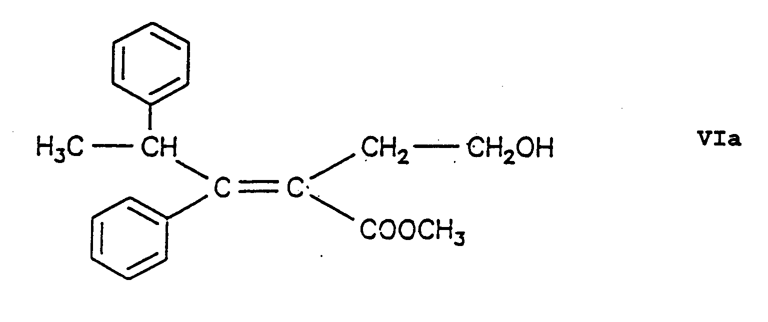 reduziert. Dieser wird vorzugsweise mit tert.Butyldimethylchlorsilan, in die entsprechende an der Hydroxymethylgruppe durch eine tert.Butyldimethylsilylgruppe geschützte Verbindung 2-[2'-(tert.Butyldimethylsilyloxy)-ethyl]-3,4-di-[phenyl]-2-pentensäuremethylester 〈2-{Phenyl-[1'-(phenyl)-ethyl]-methyliden}-4-{tert.butyldimethylsilyloxy}-buttersäuremonomethylester der Formel 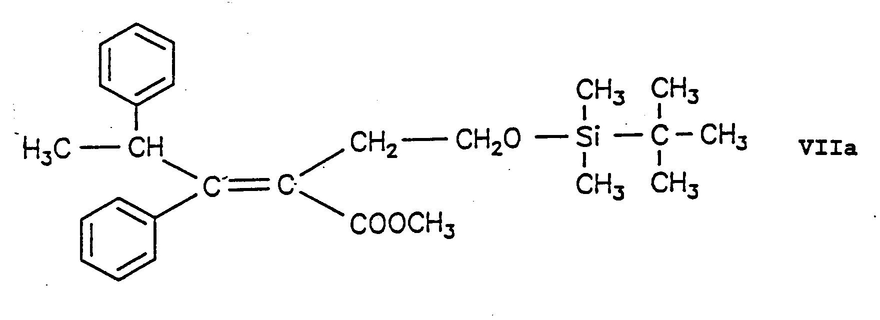 überführt. Dann erfolgt eine Kettenverlängerung um 1 Kohlenstoffatom, beispielsweise durch eine Arndt-Eistert-Reaktion oder mit Dibrommethan, zu 3-[2'-(tert.Butyldimethylsilyloxy)-ethyl]-4,5-di-[phenyl]-3-hexensäuremethylester 〈3-{Phenyl-[1'-(phenyl)-ethyl]-methyliden}-5-{tert.butyldimethylsilyloxy}-valeriansäuremonomethylester〉 der Formel

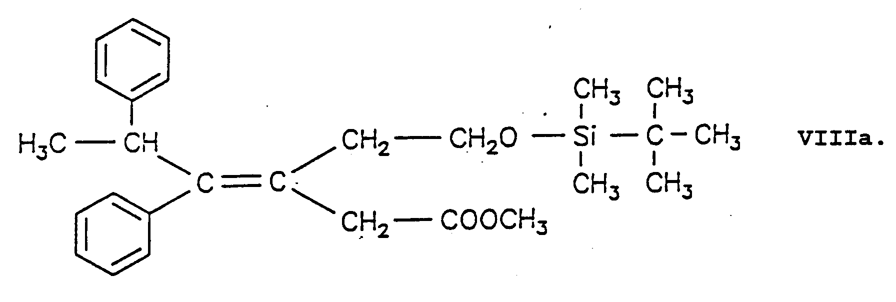

Aus dieser wird die tert.Butyldimethylsilyl-Schutzgruppe, vorteilhaft mit Tetrabutylammoniumfluorid in Tetrahydrofuran, abgespalten, wodurch 3-[2'-(Hydroxy)-ethyl]-4,5-di-[phenyl]-3-hexensäuremethylester 〈3-{Phenyl-[1'-(phenyl)-ethyl]-methyliden}-5-{hydroxy}-valeriansäuremethylester〉 der Formel 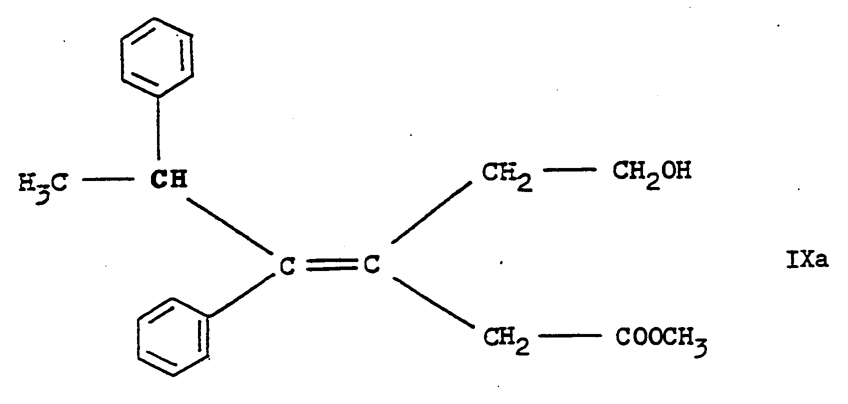 erhalten wird. Dieses wird dann zu 3-{Phenyl-[1'-(phenyl)-ethyl]-methyliden}-glutarsäuremonomethylester der Formel Ia oxydiert.

Der erfindungsgemäße 3-{Phenyl-[1'-(phenyl)-ethyl]-methyliden}-glutarsäuremonomethylester der Formel Ia kann auch durch Inkubieren von Hefezellen von Saccharomyces boulardii bei 30 bis 38°C in einem wäßrigen Hefekultur-Medium, Abtrennen der Hefezellen, Konzentrieren des zellfreien Überstandes, dessen Extrahieren mit einem Alkanol mit 4 bis 8 Kohlenstoffatomen, Eindampfen der organischen Phase, Lösen des Rückstandes in Wasser, Dialyse der erhaltenen Lösung gegen destilliertes Wasser, Konzentrieren des Dialysates, Fällen der Proteine aus diesem und Abdampfen des Fällungsmittels sowie Reinigung durch Chromatographie, wie in der unter Beanspruchung der Priorität der deutschen Patentanmeldung P 43 36 888.3 gleichzeitig eingereichten europäischen Patentanmeldung derselben Anmelderinnen beschrieben, erhalten werden, wenn die dort beschriebenen Chromatographierarbeitsgänge 2- bis 3-mal wiederholt werden, also insgesamt 4 bis 5 Chromatographierarbeitsgänge durchgeführt werden. Das so erhaltene Produkt hatte das als Fig. beigefügte Massenspektrum.

Gegenstand der Erfindung ist die Verwendung der erfindungsgemäßen {Aryl-[1'-(aryl)-alkyl]-methyliden- bzw. {Aryl-[1'-(aryl)-alkyl]-methyl}-dicarbonsäurealkylester bzw. ihrer Thiocarbonsäureesteranaloga zur Herstellung von Arzneimitteln, insbesondere Antidiarrhoika, Antibiotika, Antimykotika, Immunsuppressiva und Antitumormitteln.

Die erfindungsgemäßen Verbindungen haben nämlich gegenüber dem Stand der Technik überlegene pharmakologische Eigenschaften.

Hervorzuheben ist von der Verwendung zur Herstellung von Antidiarrhoika die zur Herstellung von solchen zur Vorbeugung und Behandlung von Sommer- und Reisedurchfall, Durchfall bei Antibiotika- und Strahlentherapie und Colitis.

Ein spezielles Beispiel für den Reisedurchfall ist die Amöbiase. Ein spezielles Beispiel für eine Diarrhoe bei Antibiotika- und Strahlentherapie, zu deren Behandlung die durch die erfindungsgemäße Verwendung erhältlichen Arzneimittel eingesetzt werden können, ist die Pseudomembrancolitis.

Die erfindungsgemäße Verwendung kann jedoch zur Herstellung von Antidiarrhoika zur Behandlung aller Arten von Diarrhoen erfolgen. Sie können nach ihrer Dauer in 4 Untertypen aufgeteilt werden:
a) akute Diarrhoe, ein selbstlimitierendes Krankheitsgeschehen, das etwa 3 Tage anhält,
b) persistierende Diarrhoe, ein Diarrhoegeschehen, das deutlich länger als 3 Tage anhält,
c) chronische Diarrhoe, ein Krankheitsgeschehen, das in der Regel mehrere Wochen bis Monate andauert und
d) chronisch rezidivierende Diarrhoe, ein Krankheitsgeschehen, das über längere Zeit schubweise erfolgt, wobei die Diarrhoeschübe durch diarrhoefreie Zeiträume voneinander getrennt sind.

Speziell die Diarrhoeformen a) und d) lassen sich weiter je nach Auslöser in verschiedenen Untertypen unterteilen; jeder dieser Untertypen ist als ein Spezialfall einer Diarrhoe anzusehen.

Eine akute Diarrhoe kann durch verschiedene Agenzien ausgelöst werden. An erster Stelle sind hier infektiöse Diarrhoen zu nennen. Die auslösenden Bakterien können unterschieden werden in nicht-invasive Bakterien, zum Beispiel Salmonellen und enterotoxische E. coli, und invasive Bakterien, zum Beispiel Shigellen, Yersinien, Campylobacter und invasive E. coli. Außerdem spielen Viren als Auslöser von akuten infektiösen Diarrhoen eine Rolle. Hier sind vor allem rotaviren- und parvovirenähnliche Organismen zu nennen. Auch Protozoen kommen als Auslöser dieser Diarrhoen in Frage, hier sind vor allen Gardia lamblia und Entamoeba histolytica zu nennen.

Akute Diarrhoen können aber auch unabhängig von lebenden Mikroorganismen hervorgerufen werden, so durch bakterielle Toxine, die mit der Nahrung aufgenommen werden, zum Beispiel Staphylococcus areus-Toxin, oder aber durch Arzneimittel wie Antibiotika, Laxantien oder Zytostatika.

Weitere Auslöser von akuten Diarrhoen können Strahlentherapie (Bestrahlung des Abdomens), aber auch psychische Belastungen (psychogene Diarrhoen) und ebenso Intoxikationen des Magen-Darm-Traktes durch toxisch wirkende Verbindungen, zum Beispiel Alkohol, Arsen und Quecksilberverbindungen, sein.

Die chronischen Diarrhoen können durch verschiedene, teilweise kombinierte Faktoren ausgelöst werden. So kann ein hyperosmotischer Darminhalt zu Malabsorptions- beziehungsweise Maldigestionsvorgängen im Darm führen, die letztlich eine erhöhte Stuhlfreguenz zur Folge haben. Daneben kann der Darm zur Hypersekretion angeregt werden, indem spezifische oder unspezifische Entzündungen im Darm stattfinden, beziehungsweise Enterotoxine oder auch Tumorerkrankungen die Sekretionsverhältnisse nachhaltig ändern. Außerdem kann der natürliche Fluß des Chymus (Nahrungsbrei) durch Veränderung der Darmmotilität, zum Beispiel peristaltische Bewegung, gestört werden, so daß auch dieses zu Diarrhoen führt. Ursachen hierfür können sein, Endokrinopathien, zum Beispiel Enzymmangelsituationen oder ein Diabetes mellitus sowie auch eine bakterielle Überwucherung (infektiöse chronische Diarrhoe). Ist das gesamte funktionelle Geschehen im Magen-Darm-Trakt gestört (Reizdarmsyndrom), so kann dieses ebenfalls mit dem Symptom Diarrhoe einhergehen. Wie auch bei der akuten Diarrhoe kann auch bei der chronischen Diarrhoe eine medikamentöse Behandlung Ursache des Symptoms sein. Auch hier sind Wirkstoffe wie Antibiotika, Laxantien und Zytostatika zu nennen, darüber hinaus aber beispielsweise auch Analgetika, Antirheumatika und Antazida. Neben diesen genannten Auslösefaktoren für eine chronische Diarrhoe gibt es verschiedene weitere, wie zum Beispiel die Krankheitsbilder Morbus Crohn und Colitis ulcerosa (chronisch entzündliche Darmerkrankung, deren Auslösefaktoren heute noch nicht endgültig geklärt sind). Ferner können Ischämie, kollagene Colitis, Divertikulose, aber auch Nahrungsmittelallergien und, wie auch bei den akuten Diarrhoen, abdominelle Bestrahlungen zu chronischen Diarrhoen führen.

Die erfindungsgemäßen Verbindungen können zusammen mit 1 oder mehr in der Pharmazie üblichen festen und/oder flüssigen Träger- und/oder Hilfsstoff(en), in Form von Arzneimittelpräparaten vorliegen. Bevorzugt sind Arzneimittelpräparate zur Anwendung.

Das erfindungsgemäße Verfahren wird an Hand der folgenden Beispiele näher erläutert.

### Beispiel 1

### a) 3-{Phenyl-[1'-(phenyl)-ethyl]-methyliden}-glutarsäuredimethylester [Formel IIIa] durch gemischte Mc Murry-Kupplung

Zu einer gerührten Suspension von 5,74 g (37,20 Millimol) Titanchlorid in 60 ml trockenem 1,2-Di-(methoxy)-ethan wurden unter Argon 0,90 g (0,13 Mol) Lithiumdraht zugegeben; anschließend wurde 1 Stunde unter Rückfluß zum Sieden erhitzt. Die nun schwarze Suspension wurde auf Raumtemperatur abkühlen gelassen und es wurde eine Lösung von 977,8 mg (4,65 Millimol) 1,2-Di-(phenyl)-propan-1-on [Formel IIa] (D. J. Cram, F. A. Abd Elhafez, J. Am. Chem. Soc. 1952, 74, 5 828 bis 5 835; A. Mc Kenzie, R. Roger, J. Chem. Soc. 1924, 125, 844 bis 854) und 809,8 mg (4,65 Millimol) handelsüblichem 3-(Oxo)-glutarsäuredimethylester {Acetondicarbonsäuredimethylester} [Formel IVa] in 10 ml trockenem 1,2-Di-(methoxy)-ethan zugesetzt. Das Reaktionsgemisch wurde 2 Stunden bei Raumtemperatur gerührt und anschließend 16 Stunden unter Rückfluß zum Sieden erhitzt. Nach dem Abkühlen wurde mit n-Pentan aufgenommen und über eine dünne Schicht von handelsüblichem Florisil® filtriert. Das Filtrat wurde unter Vakuum eingeengt und der erhaltene Rückstand säulenchromatographisch (Kieselgel, mit einer Gradientenelution mit Ether/n-Pentan steigender Polarität [O : 1OO bis 1OO : O]) gereinigt.
Ausbeute: 1,4 g (85,3%) 3-{Phenyl-[1'-(phenyl)-ethyl]-methyliden}-glutarsäuredimethylester.

### b) 3-{Phenyl-[1'-(phenyl)-ethyl]-methyliden}-glutarsäuremonomethylester [Formel Ia] durch selektive Verseifung des 3-{phenyl-[1'-(phenyl)-ethyl]-methyliden}-glutarsäuredimethylesters

1.06 g (3.00 Millimol) des wie unter a) beschriebenen erhaltenen 3-{phenyl-[1'-(phenyl)-ethyl]-methyliden}-glutarsäuredimethylesters wurden in 50 ml Ethanol und 20 ml Wasser gelöst und mit 2,4 bis 3,6 ml (0,8 bis 1,2 Äqivalenten) einer m Kaliumhydroxidlösung versetzt und 4 Stunden unter Rückfluß erhitzt. Nach Entfernen der Hauptmenge des Ethanoles unter Vakuum wurde mit Wasser aufgenommen. Zum Abtrennen von eventuell noch vorhandenem 3-{Phenyl-[1'-(phenyl)-ethyl]-methyliden}-glutarsäuredimethylester wurde 2-mal mit je 20 ml Ether ausgeschüttelt und die etherischen Phasen wurden verworfen. Dann wurde unter Eiskühlung mit konzentrierter Salzsäure angesäuert. Es wurde 5-mal mit je 50 ml Ether extrahiert. Die vereinigten organischen Phasen wurden mit wenig gesättigter Natriumchloridlösung gewaschen, über Magnesiumsulfat getrocknet und unter Vakuum vom Lösungsmittel befreit. Das rohe E/Z-Gemisch von 3-{Phenyl-[1'-(phenyl)-ethyl]-methyliden}-glutarsäuremonomethylester wurde durch Umkristallisieren oder durch Säulenchromatographie (Kieselgel, Ether/Ethylacetat steigender Polarität [O : 1OO bis 1OO : O]) gereinigt und gegebenenfalls in Isomere aufgetrennt.
Ausbeute: 0,76 g (75%) 3-{Phenyl-[1'-(phenyl)-ethyl]-methyliden}-glutarsäuremonomethylester.

### Beispiel 2

### a) 3-(Methoxy)-carbonyl-4,5-di-(phenyl)-3-hexensäure [Formel Va]

Unter Stickstoff wurde eine Suspension von 1,80 g (75,0 Millimol) Natriumhydrid in 30 ml trockenem Benzol nacheinander mit 5,26 g (25,0 Millimol) 1,2-Di-(phenyl)-propan-1-on [Formel IIa] (D. J. Cram, F. A. Abd Elhafez, J. Am. Chem. Soc. 1952, 74, 5 828 bis 5 835; A. Mc Kenzie, R. Roger, J. Chem. Soc. 1924, 125, 844 bis 854) und 10,96 g (75,0 Millimol) handelsüblichem Bernsteinsäuredimethylester [Formel IVa] versetzt. Nach Zugabe von 0,40 ml Ethanol wurde bis zum Ende der Gasentwicklung etwa 1 Stunde gerührt. Dann wurden unter Eiskühlung 5 ml Eisessig zugetropft. Anschließend wurden je 50 ml Wasser und Ether zugesetzt. Nach Abtrennen der wäßrigen Phase wurde diese 1-mal mit 20 ml Ether gewaschen. Die vereinigten etherischen Phasen wurden 5-mal mit je 50 ml 5 gew.-%-iger Natriumcarbonatlösung ausgeschüttelt, die etherische Phase wurde verworfen und der alkalische Extrakt anschließend mit Salzsäure angesäuert. Dann wurde 5-mal mit je 50 ml Ether extrahiert. Das nach Trocknen der vereinigten organischen Extrakte über Natriumsulfat und Entfernen des Lösungsmittels im Vakuum erhaltene Rohprodukt wurde durch Umkristallisieren gereinigt.
Ausbeute: 6,90 g (85%) 3-(Methoxy)-carbonyl-4,5-di-(phenyl)-3-hexensäure.

### b) 2-[2'-(Hydroxy)-ethyl]-3,4-di-[phenyl]-2-pentensäuremethylester [Formel VIa]

Es wurden 6,49 g (20,0 Millimol) wie im Abschnitt a) beschrieben erhaltene 3-(Methoxy)-carbonyl-4,5-di-(phenyl)-3-hexensäure [Formel Va] unter Stickstoff in 70 ml trockenem Tetrahydrofuran gelöst und auf -18°C abgekühlt. Dann wurden unter Rühren langsam 26,0 ml (1,3 Äqivalente) einer 1 m Lösung von Borwasserstoff in Tetrahydrofuran zugetropft. Nach Entfernen des Kältebades erwärmte sich der Ansatz auf Raumtemperatur; anschließend wurde weitere 12 Stunden gerührt. Dann wurde vorsichtig mit 50 ml Wasser versetzt und das Tetrahydrofuran unter Vakuum weitestgehend entfernt. Die verbliebene wäßrige Phase wurde 5-mal mit je 40 ml Dichlormethan extrahiert und die vereinigten Extrakte wurden über Natriumsulfat getrocknet. Der nach Einengen unter Vakuum verbliebene Rückstand wurde chromatographisch oder durch Umkristallisation gereinigt.
Ausbeute: 5,28 g (85%) 2-[2'-(Hydroxy)-ethyl]-3,4-di-[phenyl]-2-pentensäuremethylester.

### c) 2-[2'-(tert.Butyldimethylsilyloxy)-ethyl]-3,4-di-[phenyl]-2-pentensäuremethylester [Formel VIIa]

Es wurden 4,66 g (15,0 Millimol) wie im Abschnitt b) beschrieben erhaltener 2-[2'-(Hydroxy)-ethyl]-3,4-di-[phenyl]-2-pentensäuremethylester und 0,36 g (3,00 Millimol) 4-(Dimethylamino)-pyridin in 50 ml trockenem Tetrahydrofuran gelöst und 7,59 g (75,0 Millimol) Triethylamin zugegeben. Nach Zugabe von 6,78 g (45,0 Millimol) tert.Butyldimethylchlorsilan wurde das Reaktionsgemisch 24 Stunden bei Raumtemperatur gerührt. Anschließend wurde das Tetrahydrofuran unter Vakuum entfernt, es wurde mit 75 ml Ether aufgenommen und ausgeschiedenes Ammoniumsalz wurde abfiltriert. Das Filtrat wurde mit 75 ml 2 n Essigsäure ausgeschüttelt` mit Wasser gewaschen und mit gesättigter Natriumbicarbonatlösung entsäuert. Das nach Trocknen über Natriumsulfat und Entfernen des Lösungsmittels unter Vakuum erhaltene Rohprodukt wurde chromatographisch gereinigt.
Ausbeute: 5,10 g (80%) 2-[2'-(tert.Butyldimethylsilyloxy)-ethyl]-3,4-di-[phenyl]-2-pentensäuremethylester.

### d) 3-[2'-(tert.Butyldimethylsilyloxy)-ethyl]-4,5-di-[phenyl]-3-hexensäuremethylester [Formel VIIIa]

### I) Umsetzung mit 2-[2'-(tert.Butyldimethylsilyloxy)-ethyl]-3,4-di-[phenyl]-2-pentensäuremethylester unter in situ-Erzeugung von Lithiumdibrommethan

Es wurden 4,25 g (10,0 Millimol) wie im Abschnitt c) beschrieben erhaltener 2-[2'-(tert.Butyldimethylsilyloxy)-ethyl]-3,4-di-[phenyl]-2-pentensäuremethylester unter Stickstoff in 20 ml trockenem Tetrahydrofuran gelöst und mit 1,55 ml (22,0 Millimol) Dibrommethan versetzt. Das Gemisch wurde auf -78°C abgekühlt und die ebenfalls auf -78°C gekühlte Lösung von wie bei der Herstellung von Ausgangssubstanzen, Abschnitt α) beschrieben erhaltenem Lithium-2,2,6,6-tetramethylpiperidid so zugegeben, daß die Temperatur unterhalb -65°C blieb.

### II) Zugabe von Lithiumhexamethyldisilazan/Lithiumethylat und Butyllithium

Das im Abschnitt I) erhaltene Reaktionsgemisch wurde unterhalb -65°C langsam mit der Lösung von wie bei der Herstellung von Ausgangssubstanzen, Abschnitt β) beschrieben erhaltenem Lithiumhexamethyldisilazan/Lithiumethylat versetzt. Dann wurde das Kältebad entfernt und der Ansatz wurde nach Erreichen von -20°C erneut auf -78°C abgekühlt. Dann wurden 30,8 ml (40,0 Millimol) einer 1,3 m Lösung von sek.Butyllithium in Cyclohexan so zugegeben, daß die Temperatur -60°C nicht überstieg. Nach Entfernen des Kältebades wurde auf -10°C auftauen gelassen und in einem Wasserbad auf 20 bis 25°C erwärmt. Anschließend wurden 8,00 ml (20,0 Millimol) einer 2,5 m Lösung von n-Butyllithium in n-Hexan zugesetzt und 30 Minuten bei Raumtemperatur gerührt.

### III) Quenchen und Aufarbeiten

Es wurden 25 ml Acetylchlorid unter Eiskühlung und starkem Rühren zu 100 ml absolutem Ethanol getropft. Dann wurde 30 Minuten bei Raumtemperatur gerührt und erneut auf O°C abgekühlt. Das im Abschnitt II erhaltene Reaktionsgemisch wurde auf -78°C abgekühlt und langsam unter starkem Rühren zu der vorstehend beschriebenen Quench-Lösung getropft. Nach Zugabe von 200 ml Ether wurde das Gemisch mit 125 ml 10 gew.-%-iger Salzsäure gewaschen und die wäßrige Phase mit 200 ml Ether extrahiert. Die vereinigten organischen Phasen wurden mit 125 ml gesättigter Natriumchloridlösung gewaschen und über Magnesiumsulfat getrocknet. Das nach Entfernen des Lösungsmittels unter Vakuum erhaltene Rohprodukt wurde chromatographisch gereinigt.
Ausbeute: 3,51 g (80%) 3-[2'-(tert.Butyldimethylsilyloxy)-ethyl]-4,5-di-[phenyl]-3-hexensäuremethylester.

Die Herstellung von Ausgangssübstanzen ist wie folgt durchgeführt worden.

### α) Herstellung von Lithium-2,2,6,6-tetramethylpiperidid

Es wurden 4,05 ml (24,0 Millimol) 2,2,6,6-Tetramethylpiperidin unter Stickstoff zu 25 ml trockenem Tetrahydrofuran zugegeben. Dann wurden unter Eiskühlung 8,80 ml (22,0 Millimol) einer 2,5 m Lösung von n-Butyllithium in n-Hexan zugetropft. Es wurde 30 Minuten bei O°C gerührt.

### β) Herstellung von Lithiumhexamethyldisilazan/Lithiumethylat

Es wurde ein Gemisch aus 4,21 ml (20,0 Millimol) Hexamethyldisilazan, 0,59 ml (10,0 Millimol) absolutem Ethanol und 16 ml trockenem Tetrahydrofuran unter Stickstoff und Eiskühlung tropfenweise mit 12,0 ml (30 Millimol) einer 2,5 m Lösung von n-Butyllithium in n-Hexan versetzt. Nach 30 Minuten langem Rühren wurde auf -78°C abgekühlt.

### e) 3-[2'-(Hydroxy)-ethyl]-4,5-di-[phenyl]-3-hexensäuremethylester [Formel IXa]

Es wurden zu einer Lösung von 3,51 g (8,00 Millimol) wie im Abschnitt d) beschrieben erhaltenem 3-[2'-(tert.Butyldimethylsilyloxy)-ethyl]-4,5-di-[phenyl]-3-hexensäuremethylester in 15 ml trockenem Tetrahydrofuran 15,0 ml (15,0 Millimol) einer 1 m Lösung von Tetrabutylammoniumfluorid in Tetrahydrofuran zugegeben und 1 Stunde bei Raumtemperatur gerührt. Das Tetrahydrofuran wurde unter Vakuum entfernt und der Rückstand mit 150 ml Ether aufgenommen. Nach 1-maligem Waschen mit 50 ml Wasser und Trocknen über Natriumsulfat wurde das Lösungsmittel unter Vakuum entfernt. Der Rückstand wurde chromatographisch oder durch Umkristallisation gereinigt.
Ausbeute: 1,95 g (75%) 3-[2'-(Hydroxy)-ethyl]-4,5-di-[phenyl]-3-hexensäuremethylester.

### f) 3-{Phenyl-[1'-(phenyl)-ethyl]-methyliden}-glutarsäuremonomethylester [Formel Ia]

Es wurde zu 1,62 g (5,00 Millimol) wie in Abschnitt e) beschrieben erhaltenem 3-[2'-(Hydroxy)-ethyl]-4,5-di-[phenyl]-3-hexensäuremethylester in 25 ml Ether eine Lösung von 498 mg (1,67 Millimol) Natriumdichromat-dihydrat und 0,38 ml konzentrierter Schwefelsäure in 5 ml Wasser getropft. Nach 2 Stunden langem Rühren bei Raumtemperatur wurde die etherische Phase abgetrennt und die wäßrige Phase 2-mal mit je 10 ml Ether extrahiert. Die vereinigten organischen Phasen wurden über Magnesiumsulfat getrocknet und der Ether wurde unter Vakuum entfernt. Der Rückstand wurde chromatographisch oder durch Umkristallisation gereinigt.
Ausbeute: 1,18 g (70%) 3-{phenyl-[1'-(pheny1)-ethyl]-methyliden}-glutarsäuremonomethylester. Gegebenenfalls wurde das E/Z-Gemisch in Isomere aufgetrennt.

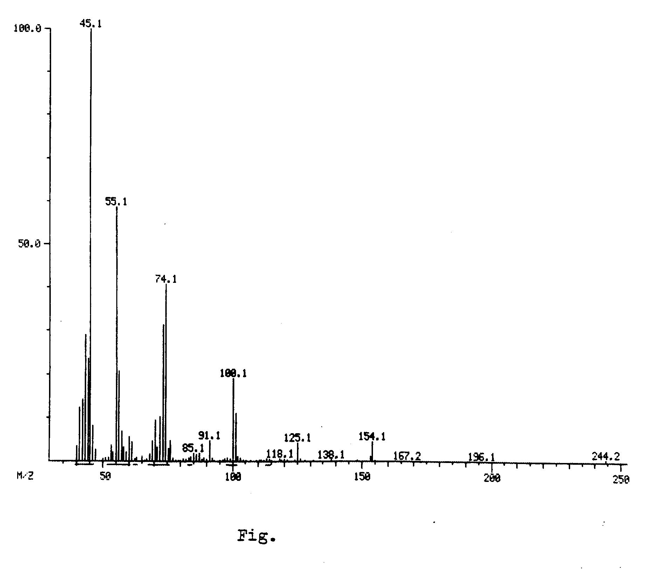

## Patentansprüche

1. {Aryl-[1'-(aryl)-alkyl]-methyliden}- bzw. {Aryl-[1'--(aryl)-alkyl]-methyl}-dicarbonsäurealkylester bzw. ihre Thiocarbonsäureesteranaloga der allgemeinen Formel 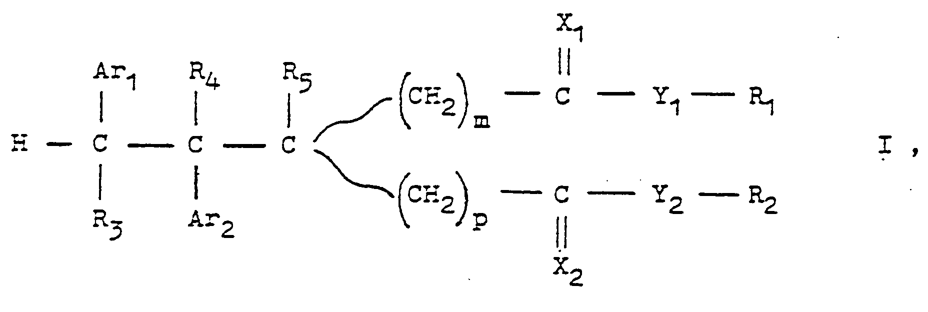 worin
Ar₁ und Ar₂ unabhängig voneinander für Arylreste stehen,
R₁ ein Wasserstoffatom oder einen Alkylrest mit 1 bis 5 Kohlenstoffatom(en) darstellt,
R₂ einen Alkylrest mit 1 bis 5 Kohlenstoffatom(en) bedeutet,
R₃ einen Alkylrest mit 1 bis 6 Kohlenstoffatom(en) darstellt,
R₄ und R₅ für Wasserstoffatome stehen oder zusammen eine weitere Bindung bedeuten,
X₁ und X₂ unabhängig voneinander für Sauerstoffbeziehungsweise Schwefelatome stehen,
Y₁ und Y₂ unabhängig voneinander Sauerstoffbeziehungsweise Schwefelatome bedeuten,
m und p unabhängig voneinander ganze Zahlen von 1 bis 8 sind und
die Wellenlinien das Erfassen sowohl der E- als auch der Z-Isomere sowie deren Mischungen versinnbildlichen,
einschließlich ihrer Z- und E-Isomere und optisch aktiven Enantiomere und deren Mischungen zur verwendung als Arzneimittel.

2. {Aryl-[1'-(aryl)-alkyl]-methyliden}- bzw. {Aryl-[1'--(aryl)-alkyl]-methyl}-dicarbonsäurealkylester bzw. ihre Thiocarbonsäureesteranaloga nach Anspruch 1, dadurch gekennzeichnet, daß die Arylreste, für welche Ar₁ und Ar₂ stehen, Phenyl- beziehungsweise Naphthylreste sind.

3. {Aryl-[1'-(aryl)-alkyl]-methyliden}- bzw. {Aryl-[1'--(aryl)-alkyl]-methyl)-dicarbonsäurealkylester bzw. ihre Thiocarbonsäureesteranaloga nach Anspruch 1 oder 2, dadurch gekennzeichnet, daß die Alkylreste, für welche R₁ stehen kann und R₂ steht, solche mit 1 bis 4, insbesondere 1 oder 2, Kohlenstoffatom(en) sind.

4. {Aryl-[1'-(aryl)-alkyl]-methyliden}- bzw. {Aryl-[1'--(aryl)-alkyl]-methyl}-dicarbonsäurealkylester bzw. ihre Thiocarbonsäureesteranaloga nach Ansprunch 1, dadurch gekennzeichnet, daß der Alkylrest, für den R₃ steht, ein solcher mit 1 bis 4, insbesondere 1 oder 2, Kohlenstoffatom(en) ist.

5. {Aryl-[1'-(aryl)-alkyl]-methyliden}- bzw. {Aryl-[1'-(aryl)-alkyl]-methyl}-dicarbonsäurealkylester bzw. ihre Thiocarbonsäureesteranaloga nach Anspruch 1, dadurch gekennzeichnet, daß m und p 1 bis 6, insbesondere 1 bis 4, sind.

6. 3-{Phenyl-[1'-(phenyl)-ethyl]-methyliden}-glutarsäuremonomethylester der Formel 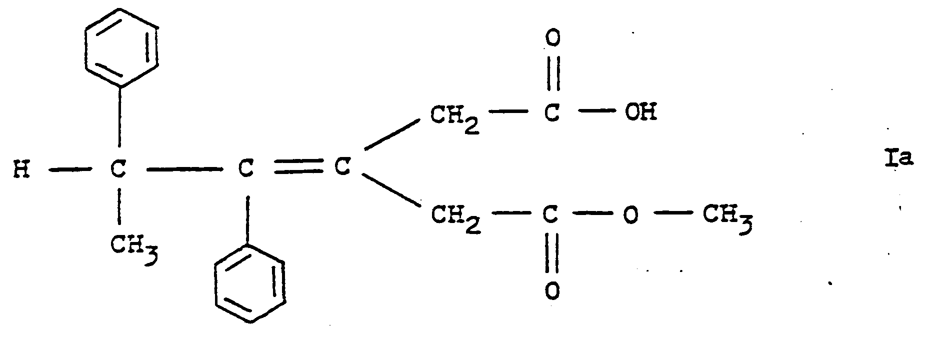 einschließlich seiner E- und Z-Isomere und optisch aktiven Enantiomere und deren Mischungen zur Verwendung als Arzneimittel.

7. Verfahren zur Herstellung der {Aryl-[1'-(aryl)-alkyl]-methyliden}- bzw. {Aryl-[1'-(aryl)-alkyl]-methyl}-dicarbonsäurealkylester bzw. ihre Thiocarbonsäureesteranaloga nach Anspruch 1 bis 6, dadurch gekennzeichnet, daß man
a) 1,2-Di-(aryl)-alkan-1-one der allgemeinen Formel 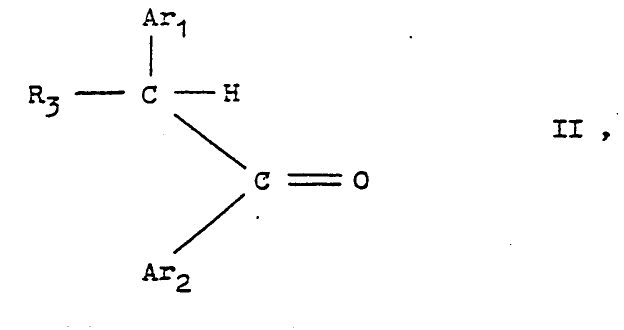 worin
Ar₁ , Ar₂ und R₃ die im Anspruch 1, 2 oder 4 angegebenen Bedeutungen haben,
mit Oxo-dicarbonsäuredialkylestern der allgemeinen Formel 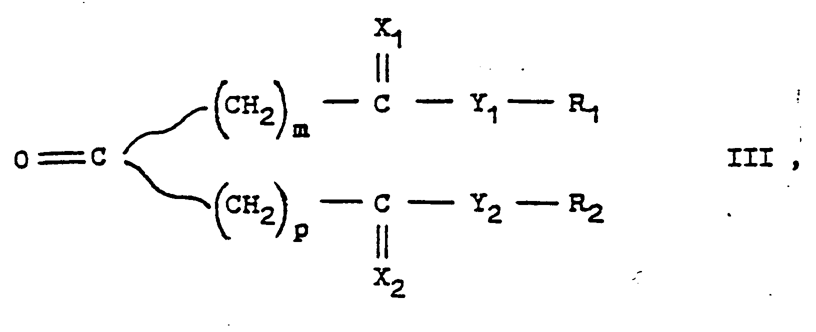 worin
X₁ , X₂ , Y₁ , Y₂ , m und p sowie die Wellenlinien die im Anspruch 1 bis 3 oder 5 angegebenen Bedeutungen haben
und
R₁ und R₂ für Alkylreste mit 1 bis 5 Kohlenstoffatom(en) stehen,
reduktiv zu {Aryl-[1'-(aryl)-alkyl]-methyliden}-dicarbonsäuredialkylestern bzw. ihren Thiocarbonsäureesteranaloga der allgemeinen Formel I, in welcher R₁ und R₂ für Alkylreste stehen und R₄ und R₅ zusammen eine weitere Bindung darstellen, kuppelt und gegebenenfalls diese letzteren regioselektiv zu {Aryl-[1'-(aryl)-alkyl]-methyliden}-dicarbonsäuremonoalkylestern bzw. ihren Thiocarbonsäureesteranaloga der allgemeinen Formel I, in welcher R₁ für ein Wasserstoffatom steht und R₂ einen Alkylrest bedeutet und R₄ und R₅ zusammen eine weitere Bindung darstellen, verseift oder/und die erhaltenen {Aryl-[1'-(aryl)-alky-1]-methyliden}-dicarbonsäuredialkylester bzw. -monoalkylester bzw. ihre Thiocarbonsäureesteranaloga der allgemeinen Formel I, in welcher R₄ und R₅ zusammen eine Doppelbindung darstellen, zu den entsprechenden {Aryl-[1'-(aryl)-alkyl]-methyl}-dicarbonsäuredialkylestern bzw. -monoalkylestern bzw. ihren Thiocarbonsäureesteranaloga der allgemeinen Formel I, in welcher R₄ und R₅ Wasserstoffatome bedeuten, hydriert
oder
b) 1,2-Di-(aryl)-alkan-1-one der allgemeinen Formel 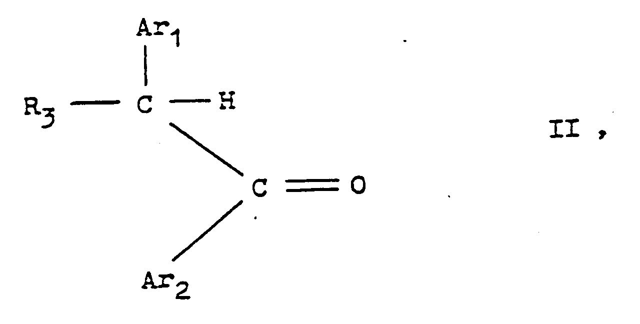 worin
Ar₁ , Ar₂ und R₃ die im Anspruch 1, 2 oder 4 angegebenen Bedeutungen haben,
mit Dicarbonsäuredialkylestern bzw- Thiocarbonsäuredialkylesteranaloga derselben der allgemeinen Formel 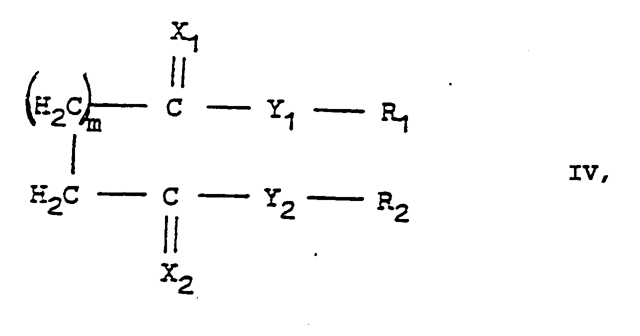 worin
X₁ , X₂ , Y₁, Y₂ und m die im Anspruch 1 oder 5 angegebenen Bedeutungen haben
und
R₁ und R₂ für Alkylreste mit 1 bis 5 Kohlenstoffatom(en) stehen,
der Stobbe-Kondensation zu 2-{Aryl-[1'-(aryl)-alkyl]-methyliden}-dicarbonsäuremonoalkylestern bzw. ihren Thiocarbonsäuremonoalkylesteranaloga der allgemeinen Formel 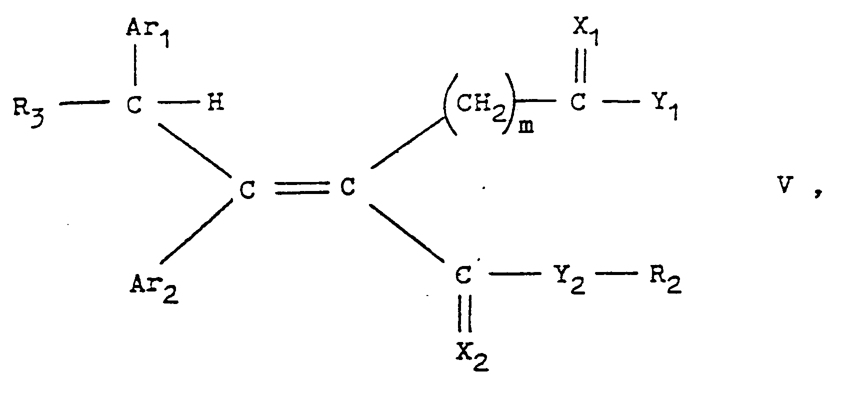 worin
Ar₁ , Ar₂ , R₃ , X₁ , X₂, Y₁ , Y₂ und m die in den Ansprüchen 1 bis 4 angegebenen Bedeutungen haben
und
R₂ für einen Alkylrest mit 1 bis 5 Kohlenstoffatom(en) steht,
unterwirft, diese letzteren selektiv zu 2-{Aryl-[1'-(aryl)-alkyl]-methyliden}-ω-(hydroxy)-carbonsäurealkylestern bzw. ihren Mercapto-, Thiocarbonsäurealkylester- bzw. Mercaptothiocarbonsäurealkylesteranaloga der allgemeinen Formel 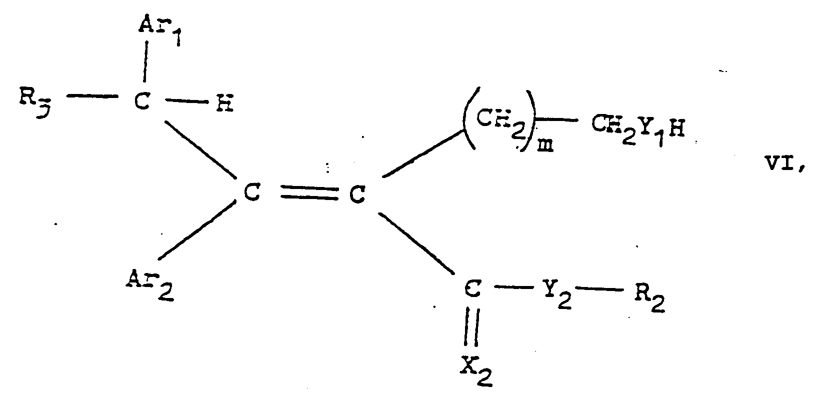 worin
Ar₁ , Ar₂ , R₃ , X₂ , Y₁ ,Y₂ und m die im Anspruch 1, 2, 4 oder 5 angegebenen Bedeutungen haben
und
R₂ für einen Alkylrest mit 1 bis 5 Kohlenstoffatom(en) steht,
reduziert, diese letzteren in die entsprechenden an der Gruppe CH₂Y₁- durch eine Trialkylsilylgruppe geschützten Verbindungen der allgemeinen Formel 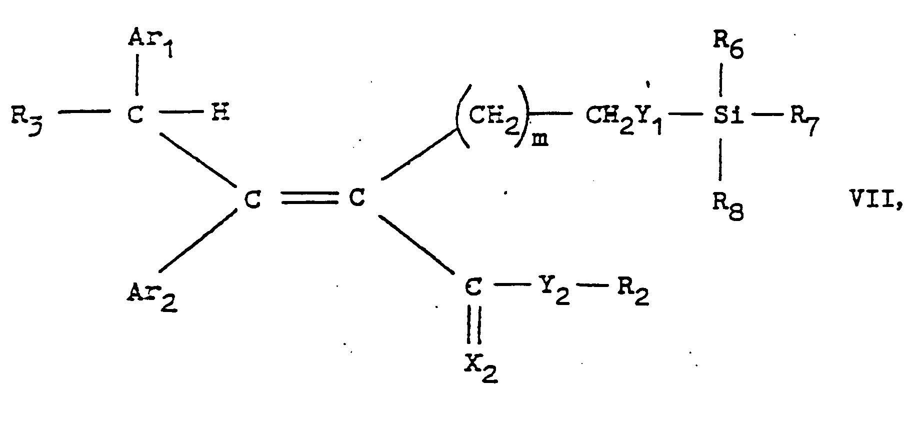 worin
Ar₁ , Ar₂ , R₃ , X₂ , Y₁ , Y₂ und m die im Anspruch 1, 2, 4 oder 5 angegebenen Bedeutungen haben
und
R₂, R₆ , R₇ und R₈ unabhängig voneinander für Alkylreste mit 1 bis 5 Kohlenstoffatom(en) stehen,
überführt, an der Alkoxycarbonylgruppe bzw. ihren Thioanaloga der letzteren Verbindungen eine Kettenverlängerung um 1 Kohlenstoffatom zu 3-{Aryl-[1'-(aryl)-alkyl]-methyliden}-ω-(trialkylsilyloxy)-carbonsäurealkylestern bzw. ihren Mercapto-, Thiocarbonsäurealkylester- bzw. Mercaptothiocarbonsäurealkylesteranaloga der allgemeinen Formel 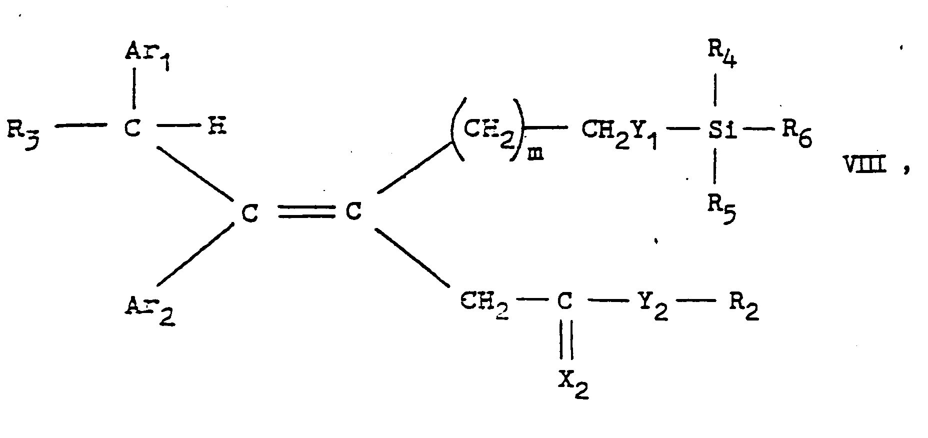 worin
Ar₁ , Ar₂ , R₃ , X₂ , Y₁ , Y₂ und m die im Anspruch 1, 2, 4 oder 5 angegebenen Bedeutungen haben
und
R₂ , R₆ , R₇ und R₈ unabhängig voneinander für Alkylreste mit 1 bis 5 Kohlenstoffatom(en) stehen,
vornimmt und gegebenenfalls durch Wiederholen der Kettenverlängerungsumsetzung weitere Kettenverlängerungen [um 1 oder mehr weitere Kohlenstoffatom(e)] durchführt, die letzteren durch Abspalten der Trialkylsilylgruppe in {Aryl-[1'-(aryl)-alkyl]-methyliden}-ω-(hydroxy)-carbonsäurealkylester bzw. ihre Mercapto-, Thiocarbonsäurealkylester- bzw. Mercaptothiocarbonsäurealkylesteranaloga der allgemeinen F 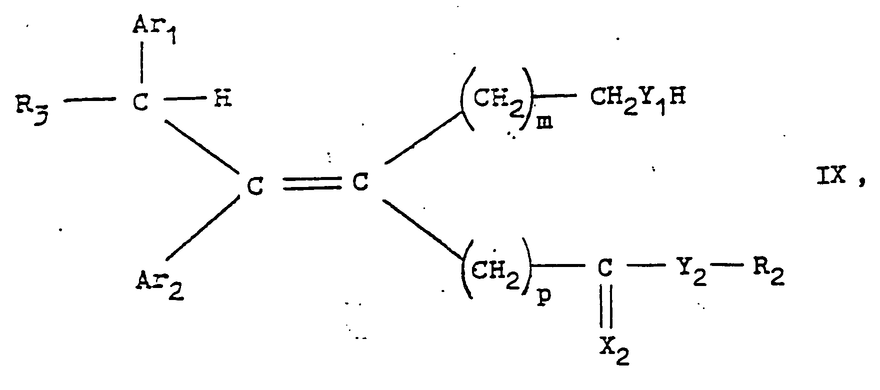 ormel worin
Ar₁ , Ar₂ , R₃ , X₂ , Y₁ , Y₂ , m und p die im Anspruch 1, 2, 4 oder 5 angegebenen Bedeutungen haben
und
R₂ , R₆ , R₇ und R₈ unabhängig voneinander für Alkylreste mit 1 bis 5 Kohlenstoffatom(en) stehen,
überführt und die letzteren oxydiert sowie gegebenenfalls die erhaltenen {Aryl-[1'-(aryl)-alkyl]--methyliden}-dicarbonsäuredialkylester bzw. -monoalkylester bzw. ihre Thiocarbonsäureesteranaloga der allgemeinen Formel I, in welcher R₄ und R₅ zusammen eine Doppelbindung darstellen, zu den entsprechenden {Aryl- [1' -(aryl)-alkyl]- methyl}-dicarbonsäuredialkylestern bzw. -monoalkylestern bzw. ihren Thiocarbonsäureesteranaloga der allgemeinen Formel I, in welcher R₄ und R₅ Wasserstoffatome bedeuten, hydriert, worauf man gegebenenfalls die erhaltenen {Aryl-[1'-(aryl)-alkyl]-methyliden}- bzw. {Aryl-[1'-(aryl)-alkyl]-methyl}-dicarbonsäurealkylester bzw. ihre Thiocarbonsäureesteranaloga der allgemeinen Formel I in ihre Isomere trennt oder die in Form von Isomeren erhaltenen {Aryl-[1'-(aryl)-alkyl]-methyliden}- bzw. {Aryl-[1'-(aryl)-alkyl]-methyl}-dicarbonsäurealkylester bzw. ihre Thiocarbonsäureesteranaloga der allgemeinen Formel I in isomere Mischungen überführt.

8. Verwendung der {Aryl-[1'-(aryl)-alkyl]-methyliden}- bzw. {Aryl-[1' -(aryl) -alkyl]-methyl}-dicarbonsäurealkylester bzw. ihrer Thiocarbonsäureesteranaloga nach Anspruch 1 bis 6 zur Herstellung von Antidiarrhoika, Antibiotika, Antimykotika, Immunsuppressiva und Antitumormitteln.

9. Verwendung nach Anspruch 8, dadurch gekennzeichnet, daß die Verwendung zur Herstellung von Antidiarrhoika eine solche zur Herstellung von solchen zur Vorbeugung und Behandlung von Sommer- und Reisedurchfall, Durchfall bei Antibiotika- und Strahlentherapie und/oder Colitis ist.

## Claims

1. The {aryl-[1'(aryl)-alkyl]-methyliden}- or {aryl-[1'-(aryl)-alkyl]-methyl} dicarboxylic acid alkyl esters or the thiocarboxylic acid ester analogues thereof having the general formula 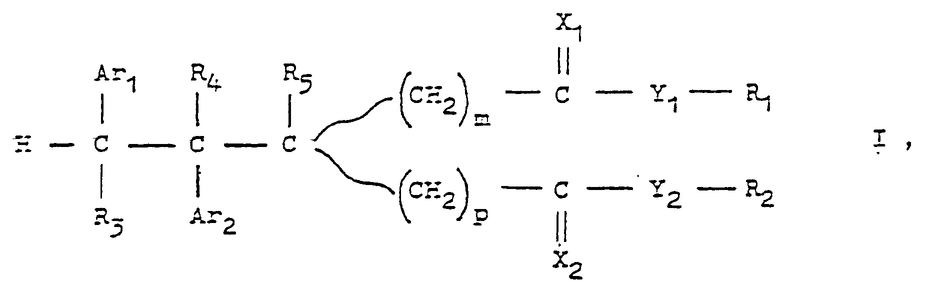 wherein
Ar₁ and Ar₂ represent, independently from each other, aryl moieties,
R₁ represents a hydrogen atom or an alkyl moiety having 1 to 5 carbon atom(s),
R₂ represents an alkyl moiety having 1 to 5 carbon atom(s),
R₃ represents an alkyl moiety having 1 to 6 carbon atom(s),
R₄ and R₅ represent hydrogen atoms or together stand for another bond,
X₁ and X₂ represent, independently from each other, oxygen or sulphur atoms,
Y₁ and Y₂ represent, independently from each other, oxygen or sulphur atoms,
m and p are, independently from each other, integers from 1 to 8, and
the wave lines illustrate the gathering of both the E and Z isomers as well as the mixtures thereof,
including the Z and E isomers and optically active enantiomers and the mixtures thereof for the use as a drug.

2. The {aryl-[1'-(aryl)-alkyl]-methyliden}- or {aryl-[1'-(aryl)-alkyl]-methyl} dicarboxylic acid alkyl ester or the thiocarboxylic acid ester analogues thereof according to Claim 1, characterized in that the aryl moieties which are represented by Ar₁ and Ar₂ are phenyl or naphthyl moieties.

3. The {aryl-[1'-(aryl)-alkyl]-methyliden}- or {aryl-[1'-(aryl)-alkyl]-methyl} dicarboxylic acid alkyl ester or the thiocarboxylic acid ester analogues thereof according to Claim 1 and Claim 2, characterized in that the alkyl moieties which can be represented by R₁ and which are represented by R₂ are those having 1 to 4, particularly 1 or 2 carbon atom(s).

4. The {aryl-[1'-(aryl)-alkyl]-methyliden}- or {aryl-[1'-(aryl)-alkyl]-methyl} dicarboxylic acid alkyl ester or the thiocarboxylic acid ester analogues thereof according to Claim 1, characterized in that the alkyl moiety which is represented by R₃ is one having 1 to 4, particularly 1 or 2 carbon atom(s).

5. The {aryl-[1'-(aryl)-alkyl]-methyliden}- or {aryl-[1'-(aryl)-alkyl]-methyl} dicarboxylic acid alkyl ester or the thiocarboxylic acid ester analogues thereof according to Claim 1, characterized in that m and p are 1 to 6, particularly 1 to 4.

6. The 3-{phenyl-[1'-(phenyl)-ethyl]-methyliden} monomethyl glutarate having the formula 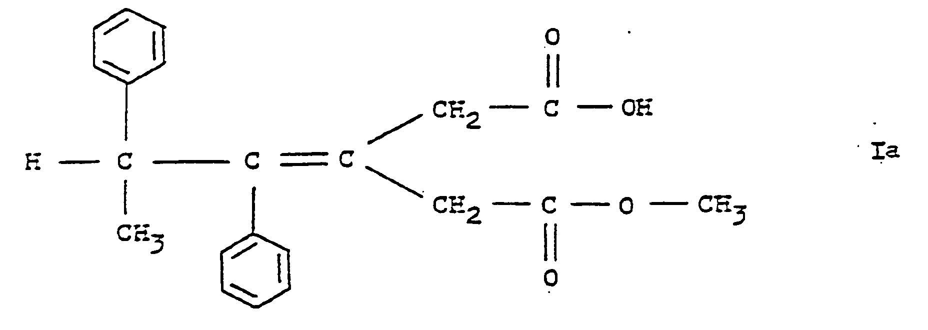 including its E and Z isomers and optically active enantiomers and the mixtures thereof for the use as a drug.

7. The process for producing the {aryl-[1'-(aryl)-alkyl]-methyliden} or {aryl-[1'-(aryl)-alkyl]-methyl} dicarboxylic acid ester or the thiocarboxylic acid ester analogues thereof according to Claims 1 to 6, characterized in that
a) 1,2-di(aryl)alkane-1-one having the formula 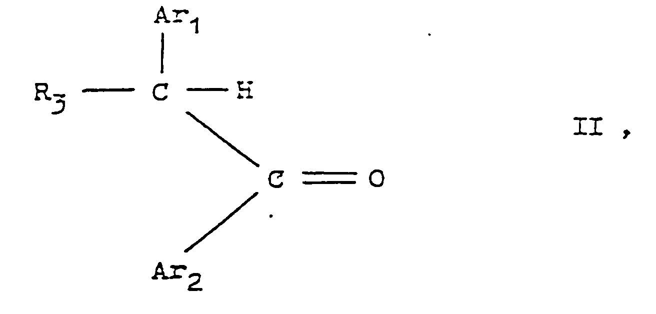 wherein
Ar₁, Ar₂ and R₃ are as defined in Claim 1, 2 or 4,
is reductively coupled with oxo dicarboxylic acid dialkyl ester having the general formula 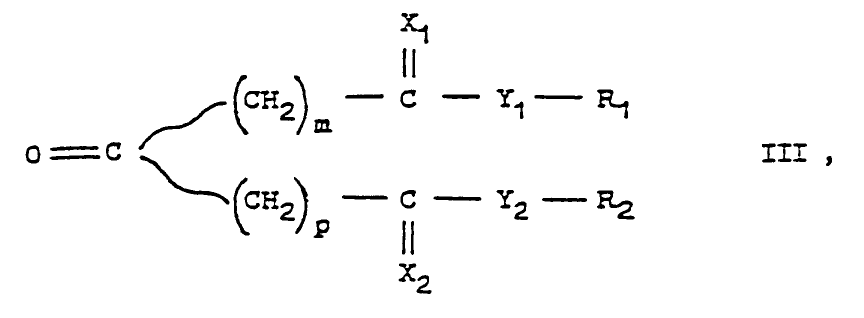 wherein
X₁, X₂, Y₁, Y₂, m and p as well as the wave lines are as defined in Claims 1 to 3 or 5
and
R₁ and R₂ represent alkyl moieties having 1 to 5 carbon atom(s),
to {aryl-[1'-(aryl)-alkyl]-methyliden} dicarboxylic acid dialkyl esters or the thiocarboxylic acid ester analogues thereof having the formula I, in which R₁ and R₂ represent alkyl moieties and R₄ and R₅ together represent another bond and optionally these latter are regioselectively saponified to {aryl-[1'-(aryl)-alkyl]-methyliden} dicarboxylic acid mono-alkyl esters or the thiocarboxylic acid ester analogues thereof having the general formula I, in which R₁ represents a hydrogen atom and R₂ represents an alkyl moiety and R₄ and R₅ together represent another bond and / or the resulting {aryl-[1'-(aryl)-alkyl]-methyliden} dicarboxylic acid dialkyl or monoalkyl esters or the thiocarboxylic acid ester analogues having the general formula I, in which R₄ and R₅ together represent a double bond, are hydrogenated to the corresponding {aryl-[1'-(aryl)-alkyl]-methyl} dicarboxylic dialkyl esters or monoalkyl esters or the thiocarboxylic acid ester analogues thereof having the general formula I, in which R₄ and R₅ represent hydrogen atoms are hydrogenated
or
b) 1,2-di(aryl)alkane-1-one having the formula 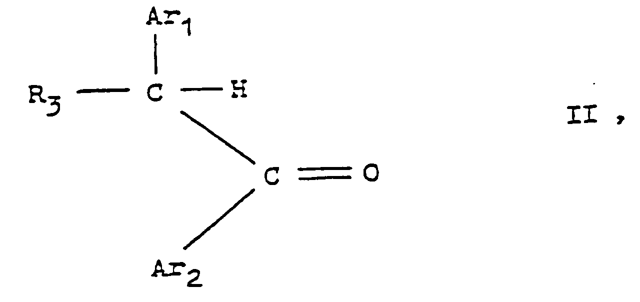 wherein
Ar₁, Ar₂ and R₃ are as defined in Claim 1, 2 or 4,
is subjected to the Stobbe-condensation with dicarboxylic acid dialkyl esters or the thiocarboxylic acid dialkyl ester analogues thereof having the general formula 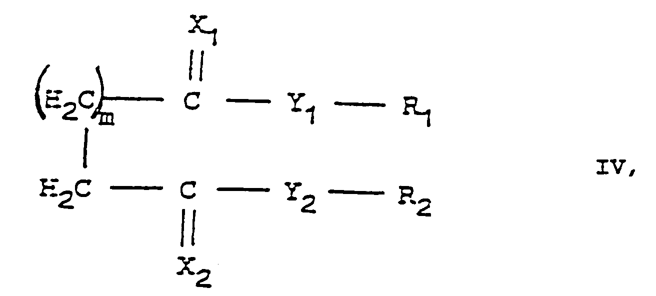 wherein
X₁, X₂, Y₁, Y₂, and m are as defined in Claims 1 or 5
and
R₁ and R₂ represent alkyl moieties having 1 to 5 carbon atom(s),
to 2-{aryl-[1'-(aryl)-alkyl]-methyliden} dicarboxylic acid monoalkyl esters or the thiocarboxylic acid monoalkyl ester analogues thereof having the formula 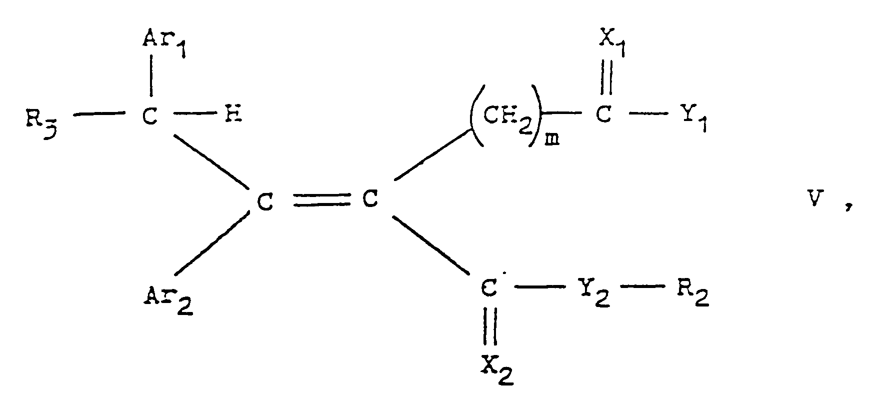 wherein
Ar₁, Ar₂, R₃, X₁, X₂, Y₁, Y₂, and m are as defined in the Claims 1 to 4,
and
R₂ represents an alkyl moiety having 1 to 5 carbon atom(s),
the latter are selectively reduced to 2-{aryl-[1'-(aryl)-alkyl]-methyliden}-ω-(hydroxy)carboxylic acid alkyl esters or the mercapto-, thiocarboxylic acid alkyl esters or mercapto thiocarboxylic acid alkyl ester analogues having the formula 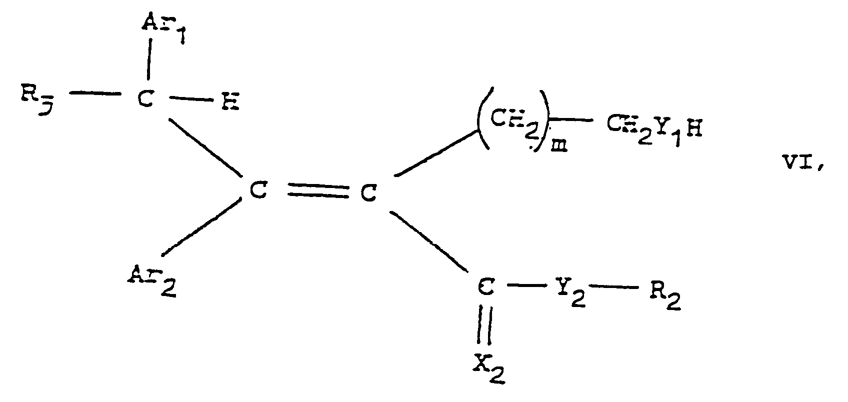 wherein
Ar₁, Ar₂, R₃, X₂, Y₁, Y₂, and m are as defined in Claim 1, 2, 4 or 5
and
R₂ represents an alkyl moiety having 1 to 5 carbon atom(s),
these latter are transformed to the corresponding compounds protected by a trialkyl silyl group at the CH₂Y₁- having the formula 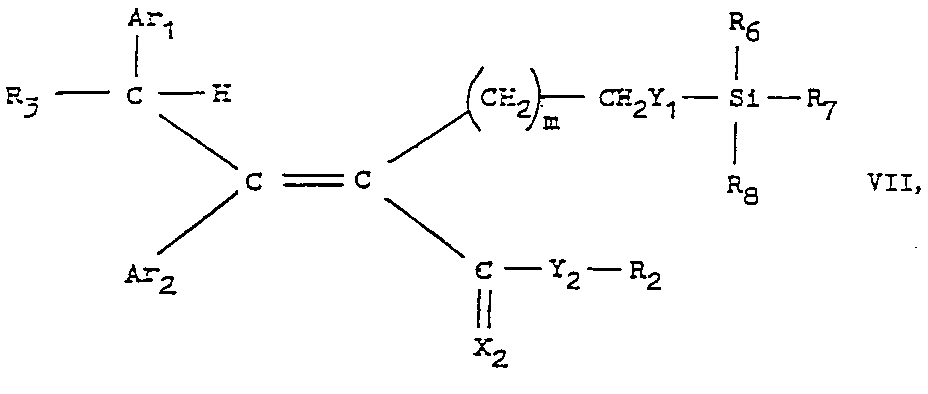 wherein
Ar₁, Ar₂, R₃, X₂, Y₁, Y₂, and m are as defined in Claim 1, 2, 4 or 5
and
R₂, R₆, R₇ and R₈ represent, independently an alkyl moiety having 1 to 5 carbon atom(s),
a chain lengthening by 1 carbon atom at the alkoxy carbonyl group or its thio analogues of the last compounds to 3-{aryl-[1'-(aryl)-alkyl]-methyliden}-ω-(trialkylsilyloxy)-carboxylic acid alkyl esters or the mercapto-, thiocarboxylic acid alkyl esters or mercapto thiocarboxylic acid alkyl ester analogues having the formula 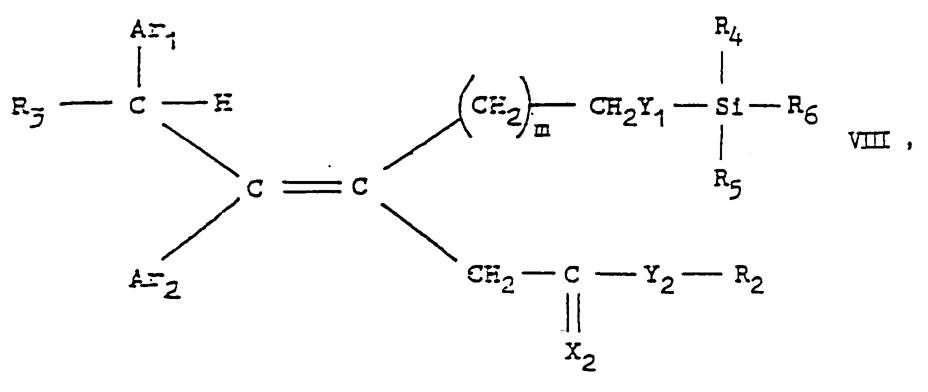 wherein
Ar₁, Ar₂, R₃, X₂, Y₁, Y₂, and m are as defined in Claim 1, 2, 4 or 5
and
R₂, R₆, R₇ and R₈ represent, independently from each other, alkyl moieties having 1 to 5 carbon atom(s),
is conducted and optionally further chain lengthenings [by 1 or more additional carbon atom(s)] are conducted by repeating the chain lengthening reaction, the latter are converted to {aryl-[1'-(aryl)-alkyl]-methyliden)-ω-(hydroxy)-carboxylic acid alkyl esters or the mercapto-, thiocarboxylic acid alkyl esters or mercapto thiocarboxylic acid all ester analogues thereof having the formula 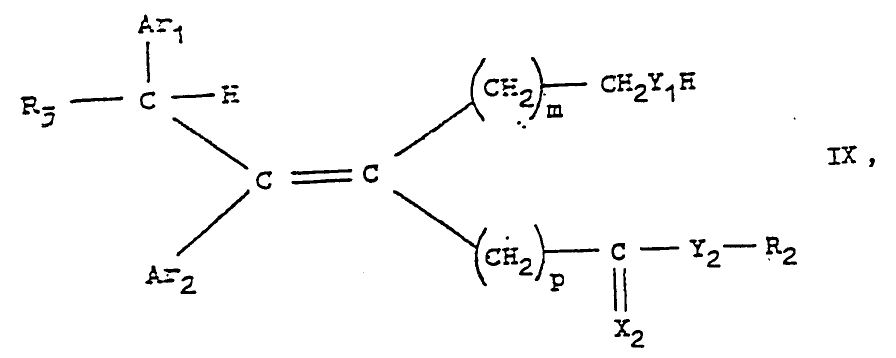 wherein
Ar₁, Ar₂, R₃, X₂, Y₁, Y₂, m and p are as defined in Claim 1, 2, 4 or 5
and
R₂, R₆, R₇ and R₈ represent, independently from each other, alkyl moieties having 1 to 5 carbon atom(s),
by cleaving the trialkyl silyl group and the latter are oxtdised and optionally the obtained {aryl-[1'-(aryl)-alkyl]-methyliden} dicarboxylic acid dialkylesters or monoalkyl esters or the thiocarboxylic acid ester analogues thereof having the general formula I, in which R₄ and R₅ together represent a double bond, are hydrogenated to the corresponding {aryl-[1'-(aryl)-alkyl]-methyl} dicarboxylic acid dialkylesters or monoalkyl esters or the thiocarboxylic acid ester analogues thereof having the general formula I, in which R₄ and R₅ represent hydrogen atoms, whereupon the resulting {aryl-[1'-(aryl)-alkyl]-methyliden} or {aryl-[1'-(aryl)-alkyl]-methyl} dicarboxylic acid alkylesters or the thiocarboxylic acid ester analogues thereof having the general formula I are optionally separated into the isomers thereof or the {aryl-[1'-(aryl)-alkyl]-methyliden} or {aryl-[1'-(aryl)-alkyl]-methyl} dicarboxylic acid alkylesters or the thiocarboxylic acid ester analogues thereof having the general formula I obtained in form of isomers are converted to isomeric mixtures.

8. The use of {aryl-[1'-(aryl)-alkyl]-methyliden}- or {aryl-[1'-(aryl)-alkyl]-methyl) dicarboxylic acid alkylesters or the thlocarboxylic acid ester analogues thereof according to Claims 1 to 6 for producing antidiarrhoeal agents, antibiotics, antimycotics, immunosuppressives and antitumor agents.

9. The use according to Claim 8, characterized in that the use for producing antidiarrhoeal agents is of same kind as for producing such for the prevention and treatment of summer and travelling diarthoea, diarrhoea occurring with antibiotic and radio therapy and / or colitis.

## Revendications

1. Esters {aryl-[1'-(aryl)-alkyl]-méthylidène}- ou (aryl-[1'-(aryl)-alkyl]-méthyl)-dicarboxylates d'alkyle et leurs analogues esters thiocarboxyliques répondant à la 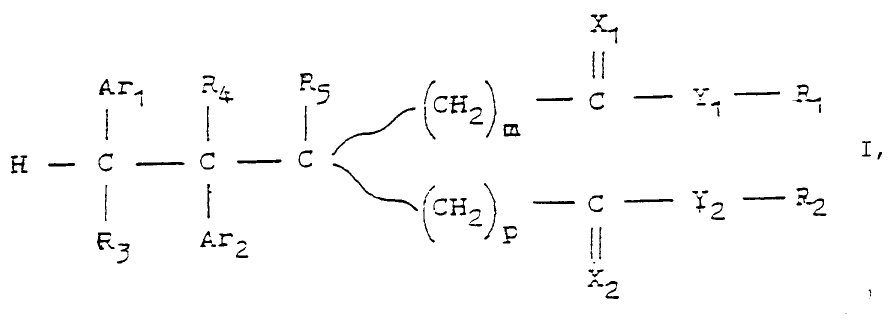 formule générale : où
Ar₁ et Ar₂ représentent indépendamment l'un de l'autre des restes aryles,
R₁ représente un atome d'hydrogène ou un reste alkyle ayant de 1 à 5 atomes de carbone,
R₂ signifie un reste alkyle ayant de 1 à 5 atomes de carbone,
R₃ représente un reste alkyle ayant de 1 à 6 atomes de carbone,
R₄ et R₅ représentent des atomes d'hydrogène ou signifient ensemble une liaison supplémentaire,
X₁ et X₂ représentent indépendamment l'un de l'autre des atomes d'oxygène ou des atomes de soufre,
Y₁ et Y₂ signifient indépendamment l'un de l'autre des atomes d'oxygène ou des atomes de soufre,
m et p sons indépendamment l'un de l'autre des nombres entiers de 1 à 8, et
les lignes ondulées symbolisent la détection des isomères E et Z, ainsi que des mélanges de ceux-ci,
y compris leurs isomères Z et E et leurs énantiomères optiquement actifs, ainsi que des mélanges de ceux-ci, destinés à être utilisés comme médicament.

2. Esters {aryl-[1'-(aryl)-alkyl]-méthylidène}- ou {aryl-[1'-(aryl)-alkyl]-méthyl}-dicarboxylates d'alkyle et leurs analogues esters thiocarboxyliques selon la revendication 1, caractérisés en ce que les restes aryles qui sont représentés par Ar₁ et Ar₂ sont des restes phényle ou naphtyle.

3. Esters {aryl-[1'-(aryl)-alkyl]-méthylidène}- ou {aryl-[1'-(aryl)-alkyl]-méthyl}-dicarboxylates d'alkyle et leurs analogues esters thiocarboxyliques selon la revendication 1 ou 2, caractérisés en ce que les restes alkyles qui peuvent être représentés par R₁ et ceux qui sont représentés par R₂ sont des groupes alkyles ayant de 1 à 4, en particulier 1 ou 2, atomes de carbone.

4. Esters {aryl-[1'-(aryl)-alkyl]-méthylidène}- ou {aryl-[1'-(aryl)-alkyl]-méthyl}-dicarboxylates d'alkyle et leurs analogues esters tniocarboxyliques selon la revendication 1, caractérisés en ce que le reste alkyle qui est représenté par R₃ est un reste alkyle ayant de 1 à 4, en particulier 1 ou 2, atomes de carbone.

5. Esters {aryl-[1'-(aryl)-alkyl]-méthylidène}- ou {aryl-[1'-(aryl)-alkyl]-méthyl}-dicarboxylates d'alkyle et leurs analogues esters thiocarboxyliques selon la revendication 1, caractérisés en ce que m et p valent de 1 à 6, en particulier de 1 à 4.

6. Ester monométhylique de l'acide 3-{phényl-[1'-(phényl)-éthyl]-méthylidène}-glutarique répondant à la formule générale : 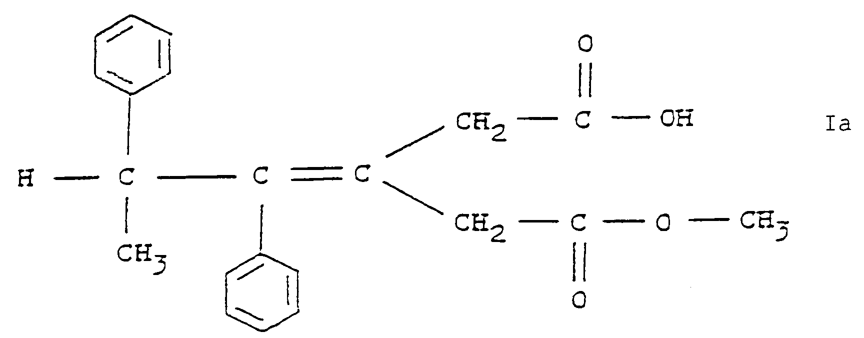 y compris ses isomères E et Z et ses énantiomères optiquement actifs, ainsi que des mélanges de ceux-ci, destinés à être utilisés comme médicament.

7. Procédé de préparation des esters {aryl-[1'-(aryl)-alkyl]-méthylidène}- ou [aryl-[1'-(aryl)-alkyl]-méthyl}-dicarboxylates d'alkyle et de leurs analogues esters thiocarboxyliques selon les revendications 1 à 6, caractérisé en ce que :
a) on couple une 1,2-di-(aryl)-alcane-1-one répondant à la formule générale : 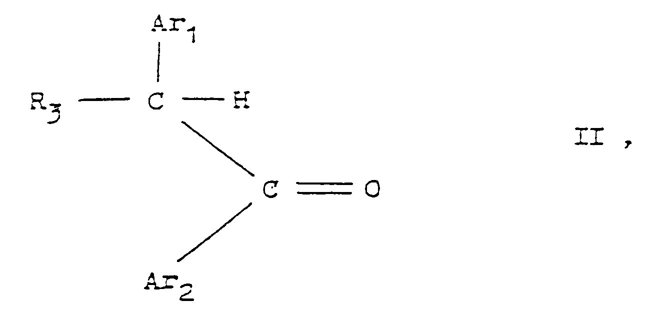 où Ar₁, Ar₂ et R₃ ont les significations indiquées dans la revendication 1, 2 ou 4,
avec des oxo-dicarboxylates de dialkyle répondant à la 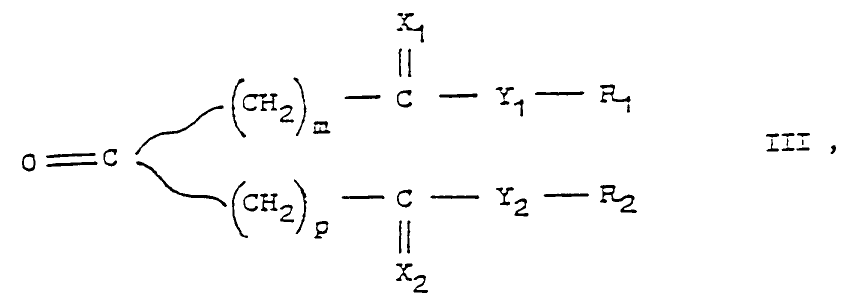 formule générale : où
X₁, X₂, Y₁, Y₂, m et p, ainsi que les lignes ondulées ont les significations indiquées dans les revendications 1 à 3 ou dans la revendication 5,
R₁ et R₂ représentent des restes alkyles ayant de 1 à 5 atomes de carbone,
qu'on transforme par réduction en esters {aryl-[1'-(aryl)-alkyl]-méthylidène}-dicarboxylates de dialkyle ou leurs analogues esters thiocarboxyliques répondant à la formule générale I dans laquelle R₁ et R₂ représentent des restes alkyles, et R₄ et R₅ pris ensemble représentent une liaison supplémentaire, et éventuellement, on saponifie ces derniers de façon régiosélective en {aryl-[1'-(aryl)-alkyl]-méthylidène}-dicarboxylates de monoalkyle ou en leurs analogues esters thiocarboxyliques répondant à la formule générale I dans laquelle R₁ représente un atome d'hydrogène et R₂ représente un reste alkyle, et R₄ et R₅ pris ensemble représentent une liaison supplémentaire, et/ou on transforme les {aryl-[1'-(aryl)-alkyl]-méthylidène}-dicarboxylates de dialkyle ou de monoalkyle ou leurs analogues esters thiocarboxyliques obtenus, répondant à la formule générale I dans laquelle R₄ et R₅ pris ensemble représentent une liaison double, par hydrogénation, en les {aryl-[1'-(aryl)-alkyl]-méthyl}-dicarboxylates de dialkyle ou de monoalkyle ou en leurs analogues esters thiocarboxyliques correspondants, répondant à la formule générale I dans laquelle R₄ et R₅ signifient des atomes d'hydrogène, ou
b) on soumet une 1,2-di-(aryl)-alcane-1-one répondant la formule générale : 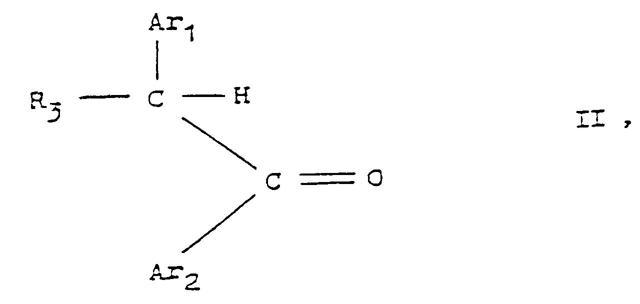 où
Ar₁, Ar₂ et R₃ ont les significations indiquées dans la revendication 1, 2 ou 4,
à la condensation de Stobbe avec des dicarboxylates de dlalkyle ou des analogues thiocarboxylates de dialkyle de ceux-ci, répondant à la formule générale : 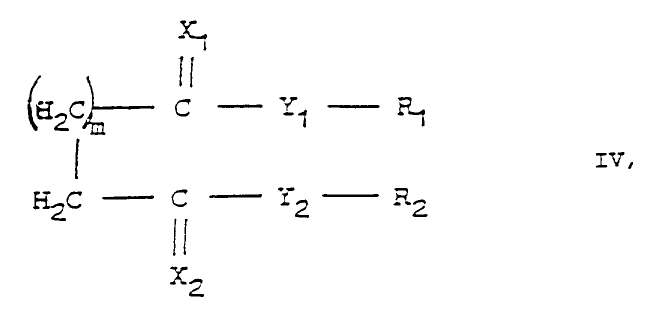 où
X₁, X₂, Y₁, Y₂ et m ont les significations indiquées dans la revendication 1 ou 5, et
R₁ et R₂ représentent des restes alkyles ayant de 1 à 5 atomes de carbone,
pour obtenir des 2-{aryl-{1'-(aryl)-alkyl]-méthylidène}-dicarboxylates de monoalkyle ou leurs analogues esters thiocarboxylates de monoalkyle, répondant à la formule générale 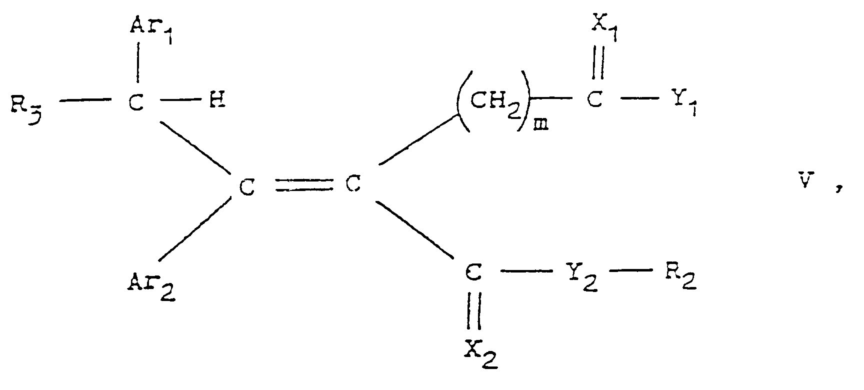 où
Ar₁, Ar₂, R₃, X₁, X₂, Y₁, Y₂ et m ont les significations indiquées dans les revendications 1 à 3, et
R₂ représente un reste alkyle ayant de 1 à 5 atomes de carbone,
on réduit ces derniers de façon sélective en 2-{aryl-[1'-(aryl)-alkyl]-méthylidène}-ω-(hydroxy)-carboxylates d'alkyle ou en leurs analogues mercapto-, thiocarboxylates d'alkyle ou mercaptothiocarboxylates d'alkyle, répondant à la formule générale : 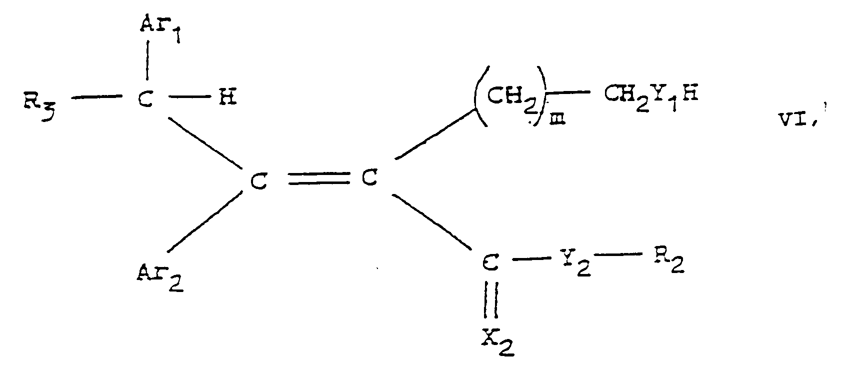 où
Ar₁, Ar₂, R₃, X₂, Y₁, Y₂ et m ont les significations indiquées dans la revendication 1, 2, 4 ou 5, et
R₂ représente un reste alkyle ayant de 1 à 5 atomes de carbone,
on transforme ces derniers en les composés correspondants protégés sur le groupe CH₂Y₁ par un groupe trialkylsilyle, répondant à la formule générale : 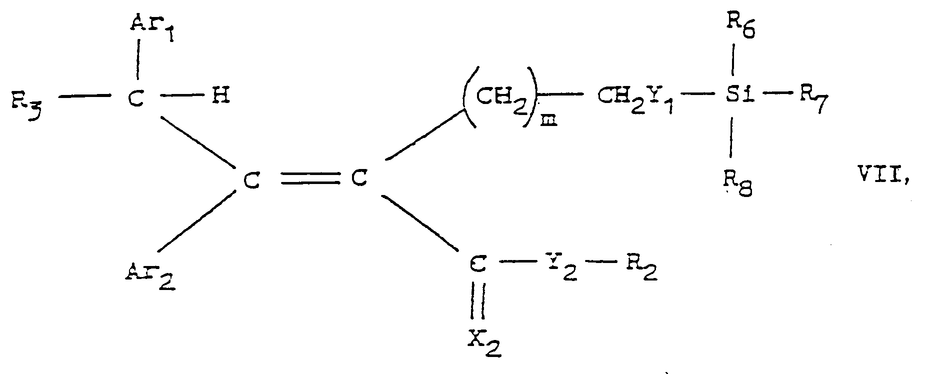 où
Ar₁, Ar₂, R₃, X₂, Y₁, Y₂ et m ont les significations indiquées dans la revendication 1, 2, 4 ou 5, et
R₂, R₆, R₇ et R₈ représentent indépendamment les uns des autres des restes alkyles ayant de 1 à 5 atomes de carbone,
sur le groupe alcoxycarbonyle ou sur les analogues thio de ces derniers composés, on procède à une augmentation de la longueur de la chaîne de 1 atome de carbone pour obtenir des 3-{aryl-[1'-(aryl)-alkyl]-méthylidène}-ω-(trialkylsilyloxy)-carboxylates d'alkyle ou leurs analogues mercapto-, thiocarboxylates d'alkyle ou mercaptothiocarboxylates d'alkyle, répondant à la formule générale : 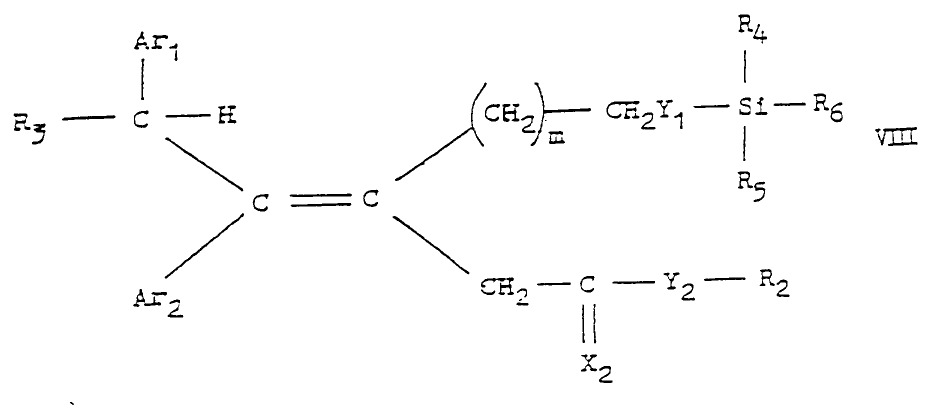 ou
Ar₁, Ar₂, R₃, X₂, Y₁, Y₂ et m ont les significations indiquées dans la revendication 1, 2, 4 ou 5, et
R₂, R₆, R₇ et R₈ représentent indépendamment les uns des autres des restes alkyles ayant de 1 à 5 atomes de carbone,
et éventuellement, en recommençant la réaction d'augmentation de la longueur de la chaîne, on effectue des augmentations supplémentaires de la longueur de la chaîne [de 1 ou plusieurs atomes de carbone supplémentaires], on transforme les composés obtenus, par clivage du groupe trialkylsilyle, en {aryl-[1'-(aryl)-alkyl]-méthylidène}-ω-(nydroxy)-carboxylates d'alkyle ou en leurs analogues mercapto-, thiocarboxylates d'alkyle ou mercaptothiocarboxylates d'alkyle, répondant à la formule générale : 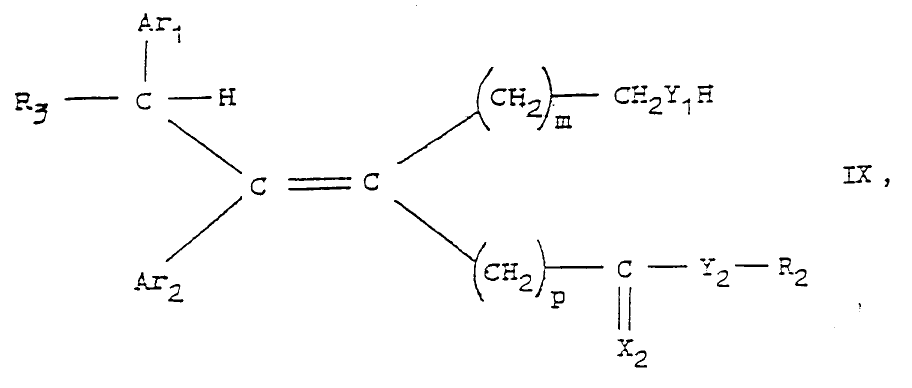 où
Ar₁, Ar₂, R₃, X₂, Y₁, Y₂, m et p ont les significations indiquées dans la revendication 1, 2, 4 ou 5, et
R₂, R₆, R₇ et R₈ représentent indépendamment les uns des autres des restes alkyles ayant de 1 à 5 atomes de carbone,
on oxyde ces derniers et éventuellement, on transforme les [aryl-[1'-(aryl)-alkyl]-méthylidène}-dicarboxylates de di-alkyle ou de monoalkyle ou leurs analogues thiocarboxylates, répondant à la formule générale I dans laquelle R₄ et R₅ pris ensemble représentent une liaison double, par hydrogénation, en [aryl-[1'-(aryl)-alkyl]-méthyl}-dicarboxylates de dialkyle ou de monoalkyle ou en leurs analogues thiocarboxylates, répondant à la formule générale I dans laquelle R₄ et R₅ signifient des atomes d'hydrogène, après quoi, éventuellement, on sépare les {aryl-[1'-(aryl)-alkyl]-méthylidène)- ou {aryl-[1'-(aryl)-alkyl]-méthyl}-dicarboxylates d'alkyle ou leurs analogues thiocarboxylates, répondant à la formule générale I, en leurs isomères, ou on transforme les {aryl-[1'-(aryl)-alkyl]-méthylidène}-ou {aryl-[1'-(aryl)-alkyl]-méthyl}-dicarboxylates d'alkyle ou leurs analogues thiocarboxylates, obtenus sous la forme d'isomères, répondant à la formule générale I, en mélanges d'isomères.

8. Utilisation des {aryl-[1'-(aryl)-alkyl]-méthylidène)- ou {aryl-[1'-(aryl)-alkyl]-méthyl}-dicarboxylates d'alkyle ou de leurs analogues esters thiocarboxylates selon les revendications 1 à 6 pour la préparation d'antidiarrhéiques, d'antibiotiques, d'agents antimycotiques, d'immunosuppresseurs et d'agents antitumoraux.

9. Utilisation selon la revendication 8, caractérisée en ce que l'utilisation pour la préparation d'antidiarrhéiques sert à préparer ceux-ci pour la prévention et le traitement de la diarrhée estivale et de la diarrhée du voyageur, de la diarrhée provoquée par un traitement aux antibiotiques et par une radiothérapie et/ou de la colite.
